# EUROPEAN PATENT APPLICATION

(11) **EP 2 177 630 A1**
(43) Date of publication of application: **21.04.2010**
(21) Application number: 08305634.1
(22) Date of filing: 02.10.2008
(51) Int. Cl.: C12Q 1/68

(54) **Methods for predicting or monitoring whether a patient affected by a cancer is responsive to a treatment with a molecule of the taxoid family**

(71) Applicant: Institut Gustave Roussy, 94800 Villejuif (FR)
(72) Inventor: Chauchereau, Anne, 92260 Fontenay-aux-Roses (FR); Al Nakouzi, Nader, 92763 Antony (FR)
(74) Representative: Gallois, Valérie

(57) **Abstract**

The present invention concerns *in vitro* methods for predicting or monitoring whether a patient affected by a cancer is responsive to a treatment with a molecule of the taxoid family based on a resistance expression signature, kits for performing the methods, and methods for screening or identifying a compound suitable for improving the treatment of a cancer with a molecule of the taxoid family or for reducing the resistance development during the treatment of a cancer with the molecule of the taxoid family

## Description

### FIELD OF THE INVENTION

The present invention relates to method for predicting the response to a treatment with a molecule of the taxoid family, kits and method for screening compounds useful for improve the treatment with the molecule.

### BACKGROUND OF THE INVENTION

Prostate cancer became, based on frequency and in Western countries, the first cancer in men, behind the lung cancer. This disease is the second cause of cancer death in men. Since 2005, more than 60,000 men are touched by prostate cancer (PCa) each year and 10,000 men died of this disease. The efficiency of docetaxel chemotherapy (Taxotere®) in prostate cancer (CaP) has been demonstrated for the first time in 2004 in two clinical trials, i.e. TAX 327 and SWOG 99-16, with an increase in survival. Accordingly, docetaxel became today a treatment of choice of metastatic hormone-refractory prostate cancers and phase III clinical trials are ongoing to assess its efficacy for the treatment of high-risk localized prostate cancer. Taxotere® is currently approved in 5 different cancer types in Europe and the US: Prostate cancer, breast cancer, lung cancer, gastric cancer and head and neck cancer. However, in spite of the survival benefit provided by this molecule, docetaxel has a great toxicity and almost half of the patients treated with docetaxel develop a resistance to the chemotherapy either from the beginning, or in a secondary way. Moreover, docetaxel is not effective on all the types of cancer. For instance, in case of breast cancer, only 30 to 50% of the metastatic tumours respond to docetaxel. Resistance to taxanes is common and there is an increasing need to try and identify those patients who will respond to treatment.

A genomic analysis was performed with two cell lines (PC3 and DU145) resistant to a docetaxel dose of 11 nM (Patterson et al, Oncogene, 2006, 25: 6113-6122). The article discloses an expression signature of 30 genes. The authors also demonstrated the effect of STAT1 and Clusterin in an *in vitro* model for the docetaxel resistance. However, the validation of the expression of these two genes in the docetaxel-resistance has not been performed on tumours. The authors further demonstrated that resveratrol leads to a decreased expression of clusterin in docetaxel resistant cells and, then to an increase of apoptosis (Sallman et al, Mol. Can. Ther., 2007, 6 : 2938-2947). Other groups used docetaxel resistance cell lines (PC3-R) in their research (Lo Nigro et al, BJU Int., 2008, 102 : 622-7). Some other groups used prostate cancer cell lines treated during a short period (24-72 h) with docetaxel for studying the role of genes in the docetaxel response.

In addition, a patent application WO 2006/062811 concerns a method for measuring resistance or sensitivity to docetaxel.

Therefore, there is still a strong need of a diagnostic method for predicting responsiveness to docetaxel and avoiding useless treatments. Indeed, before the initiation of the treatment, it is currently impossible to identify the patients who will respond to or who will have a resistance to docetaxel.

### SUMMARY OF THE INVENTION

The present invention provides an expression signature specific of the docetaxel resistance in human prostate cancer. Based on this signature, the present invention provides a method for predicting or monitoring whether a patient affected by a cancer is responsive to a treatment with a molecule of the taxoid family.

Accordingly, the present invention concerns an *in vitro* method for predicting or monitoring whether a patient affected by a cancer is responsive to a treatment with a molecule of the taxoid family, wherein the method comprises: 1) providing a biological sample from said subject; 2) determining in the biological sample the expression level of at least 5 genes selected from the group consisting of the genes listed in Tables 1 and 2, thereby predicting or monitoring whether a patient affected by a cancer is responsive to a treatment with a molecule of the taxoid family. Optionally, the method comprises determining the expression level of at least 10, 15, 20, 25, 30, 40, 50, 60, 70, 80, 90, 100, 150, 200 or 300 genes from those listed in Tables 1 and 2. Preferably, the cancer is selected from the group consisting of the breast cancer, the lung cancer, the prostate cancer, the gastric cancer and the head and neck cancer. More preferably the cancer is the prostate cancer. Preferably, the expression level is compared to a reference expression level, for instance the expression level of the genes in cell-lines or patients sensitive to the treatment by the molecule of the taxoid family. In particular, the over-expression of genes from Table 1 and/or the under-expression of genes from Table 2 are indicative of a resistance to the treatment by the molecule of the taxoid family. The expression level of genes can be determined by the quantity of protein or mRNA encoded by said genes. Preferably, the biological sample is a cancer sample.

Preferably, the at least 5 genes are selected from one of the following groups or a combination thereof:
a) RPIB9, CXCL2, AL137761, TFPI2, THC2051204, TNF, ABCB1, PURG, ADAMTS5, MCTP1, SPTLC2L, OAS3, MCTP1, GAS1, BIRC3, BQ186674, MAL, UBXD3, WNT2B, GALNT14, TM4SF1, ZAR1, A_23_P10091, GLT8D2, RXFP1, CGNL1, AK094972, LRCH2, BM930757, ATP8A1, SOX9, SLC39A8, TMEM47, SLC10A4, SLC1A3, EDG7, ITGA2, PLCXD3, BF514799, SLC16A12, THC2208430, THC2182743, C4orf18, ANKRD38, CALCRL, and LOC152573, preferably RPIB9, CXCL2, TFPI2, TNF, ABCB1, ADAMTS5, PURG, OAS3, GAS1, BIRC3, MAL, GALNT14, TM4SF1, RXFP1, ATP8A1, SOX9, SLC39A8, EDG7, ITGA2, SLC1A3, CALCRL and LOC152573;
b) RPIB9, TFPI2, ABCB1, BIRC3, WNT2B, SFRP1, FSTL1, AHR, CDKN1C, ABCB2, CYR61, WNT5A, ABCC3, JAG1, STAT1, WNT7B, CASP8, LZTS1, FZD8, GALNT14, RXFP1 and LOC152573;
c) ABCC3, CD55, COL16A1, DHRS3, FSTL1, GLS, HDL, HIVE1, LAMA2, LAMB3, LIPG, LITAF, MAL, MFHAS1, NFKBIZ, NRP1, NRP2, OAS3, OLR1, PSCDBP, RFTN1, SCARAB1, SEMA3B, SEMA3C, SFRP11, SLC1A3, ST6GAL1, TLR3, TM4SF1 and TNF;
d) ADAMTS1, ADRA2C, AKAP12, CDKN1C, CYR61, FBN1, GAS1, GPC3, IGF2, IGFBP3, JAG1, MGST1, NTN4, PDE1A, PDE4B, PDE4D, PDE4DIP, PDGFB, PHLDA1, PIM1, PPP2R2C, RGS16, SCD, SLC1A1, SMPDL3A, TFPI2 and VCAN;
e) ABCB1, AHR, AHRR, AMPH, BIRC3, CXCL2, CYP1A1, IL1R1, NQO1, PLAT, PLXNA2, SLC16A10, SLC3A1, SLC7A8, SLPI, TAP1, UGT8, UGT2B4, UGT2B7, UGT2B10, UGT2B11 and UGT2B28;
f) AQP1, ARHGDIB, BAMBI, CREB5, CXCR4, EPAS1, FGF2, FGFBP1, GRB10, IL15, MT2A, NUPR1, PDK1, PROS1, PTPN3, RPS6KA2, TFDP2, WNT2B, WNT5A and WNT7B;
g) AGT, ATP8A2, BDNF, EDG6, GAL, GATA2, ITGA2, LRP11, LZTS1, MYB, NCALD, PNOC, PTGES, SRGAP3, TAC3 and TTN;
h) AFF1, ASGR1, BLVRA, CASP8, CD40, KCNH2, NRG1, NRL, PHEX, PLAC8, SMAD7, SMAD9, SOX9, SPG20 and STAT1;
i) TNF, ABCB11, CYP1A1, AHRR, AHR, PP2R2C, ABCC3, NQO1, PIK3C3, UGT2B7, UGT2B11, UGT2B28, UGT2B4, UGT2B10, CHST7, MGST11 and UGT8; and,
j) Wnt2B, Wnt5A, Wnt7B, SFRP1, FSTL1, Jag1, Cyr61, LOC152573, FZD8 and FOXL2.

In a preferred embodiment, the molecule of the taxoid family is selected from the group consisting of docetaxel, larotaxel, XRP6258, BMS-184476, BMS-188797, BMS-275183, ortataxel, RPR 109881A, RPR 116258, NBT-287, PG-paclitaxel, ABRAXANE®, Tesetaxel, IDN 5390, Taxoprexin, DHA-paclitaxel, and MAC-321. More preferably, the molecule of the taxoid family is docetaxel.

The present invention also concerns kits and DNA chips suitable for this method. Accordingly, the present invention concerns a kit for predicting or monitoring whether a patient affected by a cancer is responsive to a treatment with a molecule of the taxoid family, wherein the kit comprises detection means selected from the group consisting of a pair of primers, a probe and an antibody specific to at least 5 genes selected from the group consisting of the genes listed in Tables 1 and 2 or a DNA chip comprising a solid support which carries nucleic acids that are specific to at least 5 genes selected from the group consisting of the genes listed in Tables 1 and 2. Preferably, the at least 5 genes of the kit or DNA chip according to the present invention are selected from one of the following groups or a combination thereof:
a) RPIB9, CXCL2, AL137761, TFPI2, THC2051204, TNF, ABCB1, PURG, ADAMTS5, MCTP1, SPTLC2L, OAS3, MCTP1, GAS1, BIRC3, BQ186674, MAL, UBXD3, WNT2B, GALNT14, TM4SF1, ZAR1, A_23_P10091, GLT8D2, RXFP1, CGNL1, AK094972, LRCH2, BM930757, ATP8A1, SOX9, SLC39A8, TMEM47, SLC10A4, SLC1A3, EDG7, ITGA2, PLCXD3, BF514799, SLC16A12, THC2208430, THC2182743, C4orf18, ANKRD38, CALCRL, and LOC152573, preferably RPIB9, CXCL2, TFPI2, TNF, ABCB1, ADAMTS5, PURG, OAS3, GAS1, BIRC3, MAL, GALNT14, TM4SF1, RXFP1, ATP8A1, SOX9, SLC39A8, EDG7, ITGA2, SLC1A3, CALCRL and LOC152573;
b) RPIB9, TFPI2, ABCB11, BIRC3, WNT2B, SFRP11, FSTL1, AHR, CDKN1C, ABCB2, CYR61, WNT5A, ABCC3, JAG1, STAT1, WNT7B, CASP8, LZTS1, FZD8, GALNT14, RXFP1 and LOC152573;
c) ABCC3, CD55, COL16A1, DHRS3, FSTL1, GLS, HDL, HIVE1, LAMA2, LAMB3, LIPG, LITAF, MAL, MFHAS1, NFKBIZ, NRP1, NRP2, OAS3, OLR1, PSCDBP, RFTN1, SCARAB1, SEMA3B, SEMA3C, SFRP11, SLC1A3, ST6GAL1, TLR3, TM4SF1 and TNF;
d) ADAMTS1, ADRA2C, AKAP12, CDKN1C, CYR61, FBN1, GAS1, GPC3, IGF2, IGFBP3, JAG1, MGST1, NTN4, PDE1A, PDE4B, PDE4D, PDE4DIP, PDGFB, PHLDA1, PIM1, PPP2R2C, RGS16, SCD, SLC1A1, SMPDL3A, TFPI2 and VCAN;
e) ABCB1, AHR, AHRR, AMPH, BIRC3, CXCL2, CYP1A1, IL1R1, NQO1, PLAT, PLXNA2, SLC16A10, SLC3A1, SLC7A8, SLPI, TAP1, UGT8, UGT2B4, UGT2B7, UGT2B10, UGT2B11 and UGT2B28;
f) AQP1, ARHGDIB, BAMBI, CREB5, CXCR4, EPAS1, FGF2, FGFBP1, GRB10, IL15, MT2A, NUPR1, PDK1, PROS1, PTPN3, RPS6KA2, TFDP2, WNT2B, WNT5A and WNT7B;
g) AGT, ATP8A2, BDNF, EDG6, GAL, GATA2, ITGA2, LRP11, LZTS1, MYB, NCALD, PNOC, PTGES, SRGAP3, TAC3 and TTN;
h) AFF1, ASGR1, BLVRA, CASP8, CD40, KCNH2, NRG1, NRL, PHEX, PLAC8, SMAD7, SMAD9, SOX9, SPG20 and STAT1;
i) TNF, ABCB11, CYP1A1, AHRR, AHR, PP2R2C, ABCC3, NQO1, PIK3C3, UGT2B7, UGT2B11, UGT2B28, UGT2B4, UGT2B10, CHST7, MGST11 and UGT8; and,
j) Wnt2B, Wnt5A, Wnt7B, SFRP1, FSTL1, Jag1, Cyr61, LOC152573, FZD8 and FOXL2.

The present invention further concerns methods for screening or identifying a compound suitable for improving the treatment of a cancer with a molecule of the taxoid family or for reducing the resistance development during the treatment of a cancer with a molecule of the taxoid family. In a first embodiment, the method comprises: 1) providing a cell-line with at least 5 genes over-expressed and/or under-expressed respectively selected from the group of over-expressed genes of Table 1 and under-expressed genes of Table 2; 2) contacting said cell-line with a test compound; 3) determining the expression level of said at least 5 genes; and, 4) selecting the compound which decreases the expression level of over-expressed genes and increases the expression level of under-expressed genes. In a second embodiment, the method comprises: 1) providing a cell-line sensitive to the molecule of the taxoid family; 2) contacting said cell-line with a test compound and the molecule of the taxoid family; 3) determining the expression level of said at least 5 genes selected from the genes listed in Tables 1 and 2; and, 4) selecting the compound which inhibits the appearance of an over-expression and/or an under-expression of at least 5 genes respectively selected from the group of genes of Table 1 and genes of Table 2. In a third embodiment, the method comprises: 1) providing a cell-line with at least on gene over-expressed and/or under-expressed respectively selected from the group consisting of RPIB9, CXCL2, AL137761, TFPI2, THC2051204, TNF, ABCB11, PURG, ADAMTS5, MCTP11, SPTLC2L, OAS3, MCTP1, GAS1, BIRC3, BQ186674, MAL, UBXD3, and WNT2B for the over-expressed genes, and GALNT14, TM4SF1, ZAR1, A_23_P10091, GLT8D2, RXFP1, CGNL1, AK094972, LRCH2, BM930757, ATP8A1, SOX9, SLC39A8, TMEM47, SLC10A4, SLC1A3, EDG7, ITGA2, PLCXD3, BF514799, SLC16A12, THC2208430, THC2182743, C4orf18, ANKRD38, CALCRL, and LOC152573 for the under-expressed genes; 2) contacting said cell-line with a test compound; 3) determining the expression level of said at least one gene; and, 4) selecting the compound which decreases the expression level of over-expressed genes and increases the expression level of under-expressed genes. Preferably, the molecule of the taxoid family is selected from the group consisting of docetaxel, larotaxel, XRP6258, BMS-184476, BMS-188797, BMS-275183, ortataxel, RPR 109881A, RPR 116258, NBT-287, PG-paclitaxel, ABRAXANE®, Tesetaxel, IDN 5390, Taxoprexin, DHA-paclitaxel, and MAC-321. More preferably, the molecule of the taxoid family is docetaxel.

### BRIEF DESCRIPTION OF THE DRAWINGS

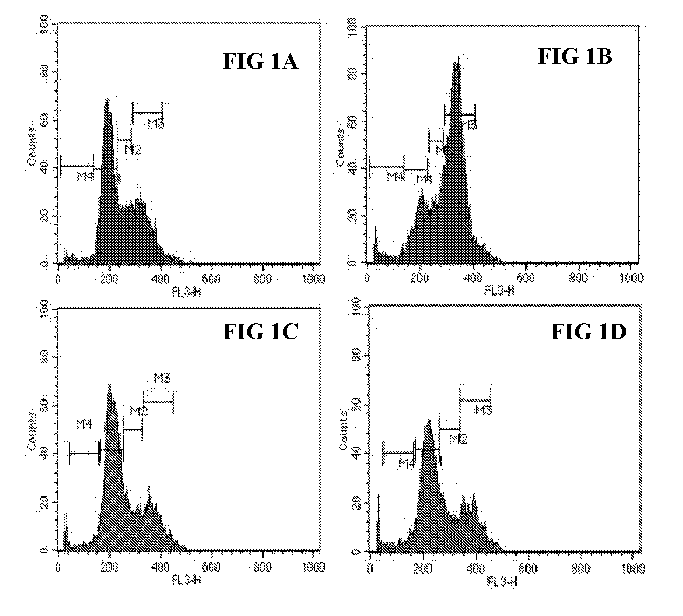

FIGURE 1 : RT PCR validation of the RPIP9 transcript. RPIP9 is the most over-expressed gene of the resistant phenotype. This gene shows a 34 fold change on the micro-array. In quantitative RT PCR, the expression of this gene showed a 450 fold ratio with the probe 1 (Taqman Applied Hs00379227_ml) and more than 1000 fold ratio with the probe 2 (Taqman Applied Hs00289927_ml). NT : without docetaxel treatment.

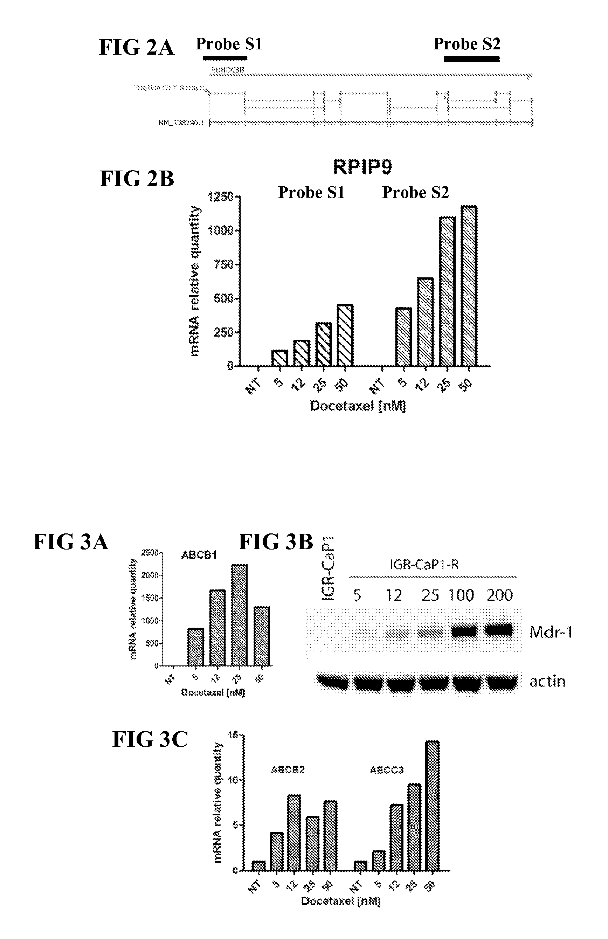

FIGURE 2 : RT PCR validation of the ABC proteins expression. ABCB1 transcript (Mdr-1) codes for P-gp1 protein which is an ATP-dependent membrane transporter responsible of cellular efflux of substances, in particular anti-tumoral drugs. This gene is frequently over-expressed in resistant phenotype. This gene belongs to the 10 most over-expressed genes in the present signature. At the highest docetaxel concentration, this gene showed a 16.22 fold change on the microarray. FIG 2A : In quantitative RT-PCR (QRT-PCR), the expression of this gene shows a ratio up to 2000 x (Taqman Applied HS00184491_ml). FIG 2B : The P-gp1 protein is also found to be over-expressed in a dose-dependent manner in resistant IGR-CaP1 cells at various doses of docetaxel. FIG 2C : The genes coding for two other proteins of the same family, i.e., ABCB2 and ABCC3, are also over-expressed, with lower fold changes. ABCB2 has a mean fold change of 3.6 on the microarray and a ratio up to 8.3 in QRT-PCR (Taqman Applied Hs00388682_ml). ABCC3 has a mean fold change of 3.1 on the microarray and a ratio up to 14.3 in QRT-PCR (Taqman Applied Hs00358656_ml).
FIGURE 3 : RT PCR validation of BIRC3 and TFPI2 gene expression. These two genes belong to the 15 most over-expressed genes in the present signature with a fold-change of 10.4 and 21.8, respectively. By QRT-PCR, the expression of these genes shows a ratio of up to 36 x (Taqman Applied Hs00154109_ml) and 64 x (Taqman Applied Hs00197918_ml), respectively.

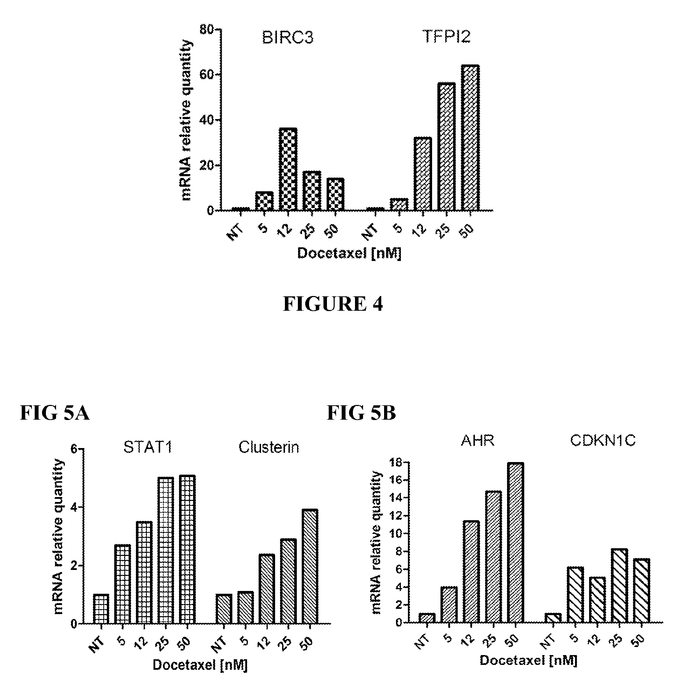

FIGURE 4 : RT PCR validation of the expression of STAT1, Clusterin, AHR and CDKN1C genes. FIG 4A : The gene encoding STAT1 shows a fold-change of 2.47 on the microarray and a fold change up to 5 by RT PCR (Taqman Applied Hs00234829_ml). The clusterin gene is slightly over-expressed in the resistant cells (Taqman Applied Hs00156548_ml). FIG 4B : Over-expression of nuclear proteins AHR (Taqman Applied Hs00907314_ml) and CDKN1C (Taqman Applied Hs00175938_ml) with a fold change on microarray of 5.4 and 5.38 on the microarray, respectively.
FIGURE 5 : RT PCR validation of under-expressed genes in the signature. GALNT14 gene (Taqman Applied Hs00226180_ml) belongs to the most under-expressed genes in the present signature with a fold-change - 10.26 on the microarray. The gene encoding Caspase 8 is also found to be under-expressed by QRT-PCR (Taqman Applied Hs01018151_ml) (Mean Fold change of - 4.51 on the microarray).

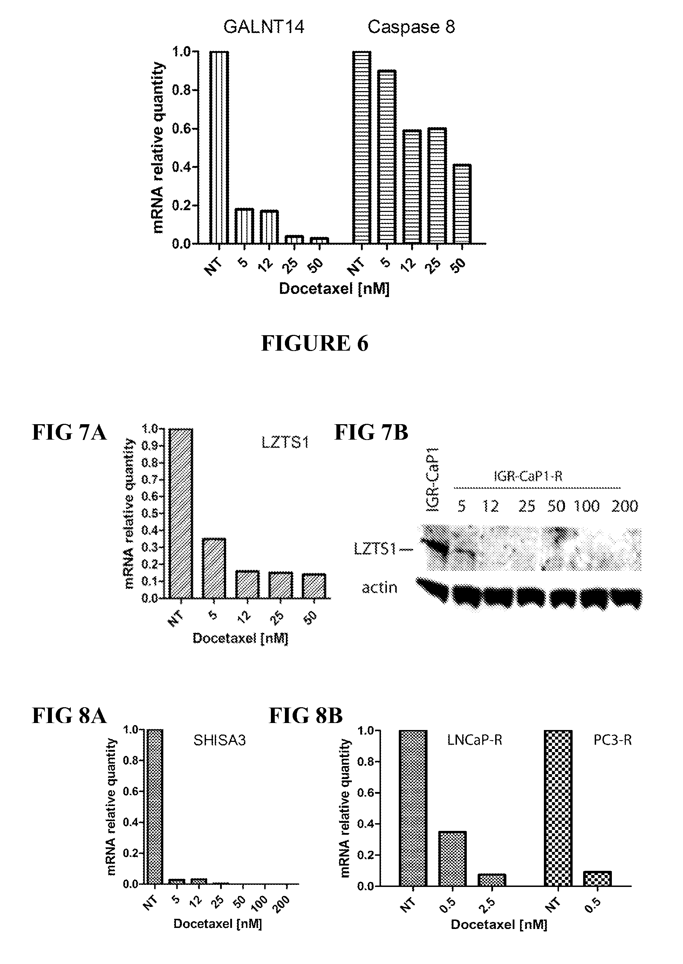

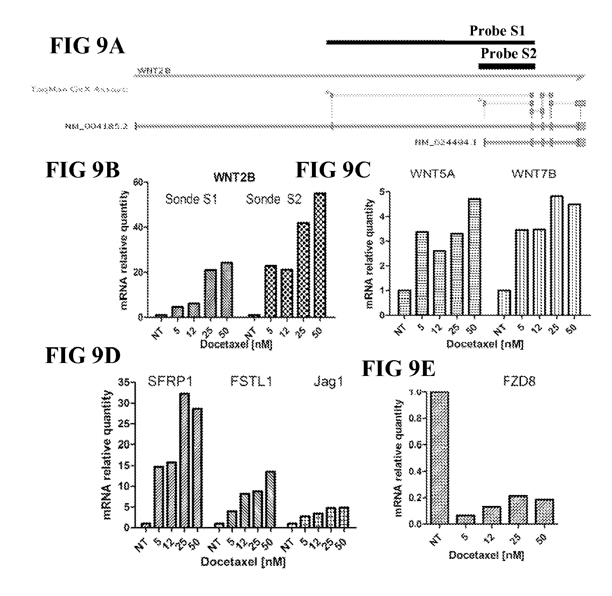

FIGURE 6 : Validation of under-expressed LZST1 gene. FIG 6A : LZST1 gene has been found to be under-expressed by QRT-PCR (Taqman Applied Hs00232762_ml) (Mean Fold change of - 4.53 on the microarray). FIG 6B : Whereas LZST1 protein is present in sensitive cells, it is absent in resistant cells at high docetaxel concentrations.
FIGURE 7 : RT PCR validation of under-expressed LOC152573 gene. FIG 7A : the LOC152573 gene encoding the human homolog of SHISA3 is the most under-expressed gene of the present signature (Mean Fold change of - 159.4 on the microarray). QRT-PCR analysis confirms this result in docetaxel resistant IGR-CaP1 cells (Taqman Applied Hs01380806_ml). FIG 7B : A strong decrease of the hSHISA gene expression has also been observed in LNCaP cells resistant to 2.5 nM of docetaxel and PC3 cells resistant to 0.5 nM of docetaxel.
FIGURE 8 : RT PCR validation of the expression of Wnt pathway genes belonging to the present signature. FIG 8A and FIG 8B : two Taqman primers were used for determining the amount of the two forms of Wnt2B (S1 : Taqman Applied Hs00244632_ml ; S2 : Taqman Applied Hs00257131_ml). FIG 8C : Genes encoding the other members of the Wnt family. Wnt5a and Wnt7b are over-expressed in a less extent (Taqman Applied Wnt5a : Hs00998537_ml ; Wnt7b : Hs00536497_ml). FIG 8D : Genes encoding other members of the Wnt pathway (Taqman Applied SFRP1 : Hs00610060_ml; FSTL1 : Hs00200053_ml; Jag1 : Hs01070032_ml). FIG 8E : The gene encoding the Frizzled 8 receptor (FDZ8) is under-expressed in docetaxel resistant cells (Taqman Applied : Hs00259040_s1).

### DETAILED DESCRIPTION OF THE INVENTION

The present invention provides the identification of protein coding genes involved in the mechanism of docetaxel resistance in prostate cancer treatment. The inventors prepared *in vitro* cellular models of docetaxel resistant prostate cancer by selecting cell clones by pharmaceutical pressure from several cellular model of prostate cancer (i.e., LNCap, PC3 and IGR-CaP1 cell lines). IGR-CaP1 cell line became resistant to increasing doses of docetaxel (0.5nM ; 5nM ; 12nM ; 25nM, 50nM ; 100nM ; 200nM). LNCaP and PC3 cell lines became resistant to docetaxel concentrations of 0.5 nM and 2.5 nM. A micro-array genomic analysis was performed by comparing sensitive and resistant IGR-CaP1 cell lines at four docetaxel concentrations (5; 12; 25 and 50 nM). This analysis led to the identification of 378 genes associated with the resistant phenotype for all the docetaxel concentrations (by 2D clusterization with a P value <10⁻¹⁰, genes with fold change > 2). In this signature, 191 genes were over-expressed and 187 genes were under-expressed. The over-expression of some signature genes was confirmed by quantitative RT-PCR (e.g., genes RPIP9; ABCB1; ABCB2; ABCC3; BIRC3; TFPI2; AHR; STAT1 ; CDKN1 ; WNT2B; WNT5A; WNT7B; SFRP1; FSTL1; Jag1) and/or by Western blot (ABCB1 protein expression). The over-expression of some genes of this signature has also been observed overexpressed by RT PCR in LNCaP cell line resistant to docetaxel concentrations of 0.5 nM and 2.5nM (e.g., ABCB1 and WNT2B genes). The under-expression of some signature genes was also confirmed by quantitative RT-PCR (e.g., genes GALNT14; LZTS1; LOC152573; FZD8) and/or by Western blot (LZTS1 protein expression). The under-expression of some genes of this signature has also been observed underexpressed by RT PCR in LNCaP cell line resistant to docetaxel concentrations of 0.5 nM and 2.5nM and PC3 cell line resistant to a docetaxel concentration of 0.5 nM (e.g., LOC152573/hSHISA3 gene). On this basis, the inventors identified a set of genes whose combined expression profiles allow to distinguish patients between responder and non-responder to a treatment with a molecule of the taxoid family. A "responder" or "responsive" patient refers to a patient who shows or will show a clinically significant recovery when treated in the cancer when treated with a molecule of the taxoid family. In particular, the size of the tumor will no more increase, decrease or the tumor will disappear.

Therefore, the present invention discloses an expression signature useful for *in vitro* method for predicting whether a patient suffering of a cancer would be responsive to a treatment with a molecule of the taxoid family. The method comprises determining the expression level of genes from the present expression signature (see Tables 1 and 2) in a biological sample of said patient. In particular, the method comprises determining the expression level of at least 5 genes of Tables 1 and 2 in a biological sample of said patient. Preferably, the method comprises determining the expression level of at least 6, 7, 8, 9, 10, 15, 20, 25, 30, 40, 50, 60, 70, 80, 90, 100, 150, 200 or 300 genes of Tables I and 2. Alternatively, the method comprises determining the expression level of 5 to 378 genes of Tables 1 and 2, optionally of 7 to 370, 8 to 360, 9 to 350, 10 to 325, 15 to 300, 20 to 250, 30 to 200, 40 to 150, 50 to 100, 60 to 90 or 70 to 80.

By "predicting" or "prediction" is intended herein the likelihood that a patient will respond or not to a molecule of the taxoid family and also the extent of the response. Predictive methods of the invention can be used clinically to make treatment decisions by choosing the most appropriate treatment modalities for any particular patient. Therefore, the present invention also concerns a method for selecting a patient suffering of a cancer for a treatment with a molecule of the taxoid family, comprising determining the expression level of at least 5, 6, 7, 8, 9, 10, 15, 20, 25, 30, 40, 50, 60, 70, 80, 90, 100, 150, 200 or 300 genes of Tables I and 2 in a biological sample of said patient and selecting the patient predicted to be responsive to a treatment with a molecule of the taxoid family.

In a first embodiment, the genes are selected from Tables 1 and 2 on the criteria of "fold change". Accordingly, the genes with the greatest fold change (in absolute value) are chosen. For instance, the genes associated with a fold change greater (in absolute value) than 2, preferably than 3, 4, 5, 6, 7, 8, 9 or 10, are selected. In a particular embodiment, the genes are selected from the group consisting of RPIB9, CXCL2, AL137761, TFPI2, THC2051204, TNF, ABCB1, PURG, ADAMTS5, MCTP1, SPTLC2L, OAS3, MCTP1, GAS1, BIRC3, BQ186674, MAL, UBXD3, WNT2B, GALNT14, TM4SF1, ZAR1, A_23_P10091, GLT8D2, RXFP1, CGNL1, AK094972, LRCH2, BM930757, ATP8A1, SOX9, SLC39A8, TMEM47, SLC10A4, SLC1A3, EDG7, ITGA2, PLCXD3, BF514799, SLC16A12, THC2208430, THC2182743, C4orf18, ANKRD38, CALCRL, and LOC152573, preferably from the group consisting of RPIB9, CXCL2, TFPI2, TNF, ABCB1, ADAMTS5, PURG, OAS3, GAS1, BIRC3, MAL, GALNT14, TM4SF1, RXFP1, ATP8A1, SOX9, SLC39A8, EDG7, ITGA2, SLC1A3, CALCRL and LOC152573. Alternatively, the genes can be selected among the genes validated by RT-PCR, in particular in the group consisting of RPIB9, TFPI2, ABCB1, BIRC3, WNT2B, SFRP1, FSTL1, AHR, CDKN1C, ABCB2, CYR61, WNTSA, ABCC3, JAG1, STAT1, WNT7B, CASP8, LZTS1, FZD8, GALNT14, RXFP1 and LOC152573.

In a second embodiment, the genes are selected from Tables 1 and 2 on the criteria of a network, that is to say that the genes are selected in one particular network. Accordingly, the genes can be selected in the group consisting of one of the following networks or a combination thereof comprising:
1) ABCC3, CD55, COL16A1, DHRS3, FSTL1, GLS, HDL, HIVE1, LAMA2, LAMB3, LIPG, LITAF, MAL, MFHAS1, NFKBIZ, NRP1, NRP2, OAS3, OLR1, PSCDBP, RFTN1, SCARAB1, SEMA3B, SEMA3C, SFRP1, SLC1A3, ST6GAL1, TLR3, TM4SF1 and TNF;
2) ADAMTS1, ADRA2C, AKAP12, CDKN1C, CYR61, FBN1, GAS1, GPC3, IGF2, IGFBP3, JAG1, MGST1, NTN4, PDE1A, PDE4B, PDE4D, PDE4DIP, PDGFB, PHLDA1, PIM1, PPP2R2C, RGS16, SCD, SLC1A1, SMPDL3A, TFPI2 and VCAN;
3) ABCB1, AHR, AHRR, AMPH, BIRC3, CXCL2, CYP1A1, IL1R1, NQO1, PLAT, PLXNA2, SLC16A10, SLC3A1, SLC7A8, SLPI, TAP1, UGT8, UGT2B4, UGT2B7, UGT2B10, UGT2B11 and UGT2B28;
4) AQP1, ARHGDIB, BAMBI, CREB5, CXCR4, EPAS1, FGF2, FGFBP1, GRB10, IL15, MT2A, NUPR1, PDK1, PROS1, PTPN3, RPS6KA2, TFDP2, WNT2B, WNT5A and WNT7B;
5) AGT, ATP8A2, BDNF, EDG6, GAL, GATA2, ITGA2, LRP11, LZTS1, MYB, NCALD, PNOC, PTGES, SRGAP3, TAC3 and TTN; and
6) AFF1, ASGR1, BLVRA, CASP8, CD40, KCNH2, NRG1, NRL, PHEX, PLAC8, SMAD7, SMAD9, SOX9, SPG20 and STAT1.

In a third embodiment, the genes are selected from Tables 1 and 2 on the criteria of their belonging to the signaling pathway of xenobiotic metabolism. Accordingly, the genes can be for instance selected from the group consisting of TNF, ABCB1, CYP1A1, AHRR, AHR, PP2R2C, ABCC3, NQO1, PIK3C3, UGT2B7, UGT2B11, UGT2B28, UGT2B4, UGT2B10, CHST7, MGST1 and UGT8. In another embodiment, the genes are selected from Tables 1 and 2 because of their membership to the Wnt pathway. Accordingly, the genes can be for instance selected from the group consisting of Wnt2B, Wnt5A, Wnt7B, SFRP1, FSTL1, Jag1, Cyr61, LOC152573, FZD8 and FOXL2.

Of course, the genes can also be selected from a combination of these particular groups.

The method can comprise the step of comparing the expression levels of the genes determined in the sample to reference or control expression levels. The reference or control expression levels are determined with a sample of cells, preferably cancer cells, which are sensitive to the molecule of the taxoid family. Alternatively, reference or control expression levels are determined with a sample of patients or subjects sensitive to the treatment with the molecule of the taxoid family. Hence, an over-expressed gene herein refers to a gene having an increased expression in comparison to the expression level of this gene in a sensitive cell, and an under-expressed gene herein refers to a gene having a decreased expression in comparison to the expression level of this gene in a sensitive cell. However, the man skilled in art understands that other references can be used. For instance, the invention also contemplates a reference level corresponding to the expression level in a cell resistant to the molecule of the taxoid family.

In particular, when the genes selected from the Table 1 are over-expressed, one can predict that the patient would be resistant to a treatment with a molecule of the taxoid family. On the contrary, when the genes selected from the Table 1 are not over-expressed, one can predict that the patient would be responsive to a treatment with a molecule of the taxoid family. At the opposite, when the genes selected from the Table 2 are under-expressed, one can predict that the patient would be resistant to a treatment with a molecule of the taxoid family. On the contrary, when the genes selected from the Table 2 are not under-expressed, one can predict that the patient would be responsive to a treatment with a molecule of the taxoid family.

In addition, the genes can be selected in such a way that they comprise some over-expressed genes and some under-expressed ones. In this embodiment, the selected genes can comprise at least 5, 6, 7, 8, 9, 10, 15, 20, 25, 30, 40, 50, 60, 70, 80, 90, 100, or 150 genes of Table 1 and at least 5, 6, 7, 8, 9, 10, 15, 20, 25, 30, 40, 50, 60, 70, 80, 90, 100, or 150 genes of Table 2. Alternatively, they can be selected in such a way that they comprise only over-expressed or under-expressed genes. In a preferred embodiment, the genes are selected among the genes having the greatest fold change.

In addition to the genes selected from Tables 1 and 2, the method can also comprise the determination of the expression level for control genes. The control genes are chosen among the genes known to have a constant expression level, in particular between sensitive and resistant cells to a molecule of the taxoid family. In addition, the expression level of at least one control gene is determined in order to normalize the result. For instance, the control gene can be GAPDH, 18S RNA, beta-actine or lamin.

The molecule of the taxoid family refers to a class of anti-tumoral drugs belonging to the taxane family. It can be selected from the group consisting of paclitaxel, docetaxel and analogs, prodrugs or formulations thereof. In particular, analogs, prodrugs or formulations thereof can be for instance selected in the group consisting of larotaxel (also called XRP9881; Sanofi-Aventis), XRP6258 (Sanofi-Aventis), BMS-184476 (Bristol-Meyer-Squibb), BMS-188797 (Bristol-Meyer-Squibb), BMS-275183 (Bristol-Meyer-Squibb), ortataxel (also called IDN 5109, BAY 59-8862 or SB-T-101131 ; Bristol-Meyer-Squibb), RPR 109881A (Bristol-Meyer-Squibb), RPR 116258 (Bristol-Meyer-Squibb), NBT-287 (TAPESTRY), PG-paclitaxel (also called CT-2103, PPX, paclitaxel poliglumex, paclitaxel polyglutamate or Xyotax^{™}), ABRAXANE® (also called Nab-Paclitaxel ; ABRAXIS BIOSCIENCE), Tesetaxel (also called DJ-927), IDN 5390 (INDENA), Taxoprexin (also called docosahexanoic acid-paclitaxel ; PROTARGA), DHA-paclitaxel (also called Taxoprexin®), and MAC-321 (WYETH). Also see the review of Hennenfent & Govindan (2006, Annals of Oncology, 17, 735-749). In a preferred embodiment of the present invention, the molecule of the taxoid family is the docetaxel.

The expression level of the selected genes can be determined by measuring the amounts of RNA, in particular mRNA, DNA, in particular cDNA, or protein using a variety of techniques well-known by the man skilled in art.

The cancer can be selected from the group consisting of the breast cancer, the lung cancer, the prostate cancer, the gastric cancer and the head and neck cancer. In a preferred embodiment, the cancer is the prostate cancer.

The term "biological sample" means any biological sample derived from a patient, preferably a sample which contains nucleic acids or proteins. Examples of such samples include fluids, tissues, cell samples, organs, biopsies, etc. Most preferred samples are cancer tissue samples, in particular breast, lung, prostate, stomach, ovary or head and neck tumor samples. Blood, plasma, saliva, urine, seminal fluid, etc, may also be used. Cancer cells obtain form blood as circulating tumor cells may also be used. The biological sample may be treated prior to its use, e.g. in order to render nucleic acids or proteins available. Techniques of cell lysis, concentration or dilution of nucleic acids or proteins, are known by the skilled person.

Generally, the expression level as determined is a relative expression level (mRNA or protein).

More preferably, the determination comprises contacting the sample with selective reagents such as probes, primers or ligands, and thereby detecting the presence, or measuring the amount, of proteins or nucleic acids of interest originally in the sample. Contacting may be performed in any suitable device, such as a plate, microtiter dish, test tube, well, glass, column, and so forth. In specific embodiments, the contacting is performed on a substrate coated with the reagent, such as a nucleic acid array or chip or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The contacting may be made under any condition suitable for a detectable complex, such as a nucleic acid hybrid or an antibody-antigen complex, to be formed between the reagent and the nucleic acids or proteins of the sample.

In a preferred embodiment, the expression level may be determined by determining the quantity of mRNA.

Methods for determining the quantity of mRNA are well known in the art. For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous.

Other methods of Amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate). For instance, the probes and primers can be selected from the Taqman Applied ones cited in the present application.

The nucleic acid primers or probes used herein may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In another preferred embodiment, the expression level is determined by DNA chip analysis. Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, *e.g.* by using radioactive or fluorescent labelling. Many variants of the microarray hybridization technology are available to the man skilled in the art (see e.g. the review by Hoheisel, et 2006)

Other methods for determining the expression level of said genes include the determination of the quantity of proteins encoded by said genes.

Such methods comprise contacting a biological sample with a binding partner capable of selectively interacting with a marker protein present in the sample. The binding partner is generally an antibody, that may be polyclonal or monoclonal, preferably monoclonal.

The presence of the protein can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the antigen and the antibody or antibodies reacted therewith.

The aforementioned assays generally involve separation of unbound protein in a liquid phase from a solid phase support to which antigen-antibody complexes are bound. Solid supports which can be used in the practice of the invention include substrates such as nitrocellulose (*e. g.*, in membrane or microtiter well form); polyvinylchloride (*e. g*., sheets or microtiter wells); polystyrene latex (*e.g.*, beads or microtiter plates); polyvinylidine fluoride; diazotized paper; nylon membranes; activated beads, magnetically responsive beads, and the like.

More particularly, an ELISA method can be used, wherein the wells of a microtiter plate are coated with an antibody against the protein to be tested. A biological sample containing or suspected of containing the marker protein is then added to the coated wells. After a period of incubation sufficient to allow the formation of antibody-antigen complexes, the plate(s) can be washed to remove unbound moieties and a detectably labeled secondary binding molecule added. The secondary binding molecule is allowed to react with any captured sample marker protein, the plate washed and the presence of the secondary binding molecule detected using methods well known in the art.

The invention further provides a tool for implementing said methods, e.g. a DNA chip comprising a solid support which carries nucleic acids that are specific to at least 5, 6, 7, 8, 9, 10, 15, 20, 25, 30, 40, 50, 60, 70, 80, 90, 100, 150, 200 or 300 genes selected from the group consisting of the genes listed in Tables 1 and 2. The DNA chip can further comprise nucleic acids for control gene, for instance a positive and negative control or a nucleic acid for an ubiquitous gene in order to normalize the results. In addition, the present invention also provides a kit for implementing said methods comprising detection means that are specific to at least 5, 6, 7, 8, 9, 10, 15, 20, 25, 30, 40, 50, 60, 70, 80, 90, 100, 150, 200 or 300 genes selected from the group consisting of the genes listed in Tables 1 and 2. In particular, the detection means can be a pair of primers, a probe or an antibody. The kit can further comprise control reagents and other necessary reagents.

In a particular embodiment, the genes are selected for the tool or kit as above detailed for the methods of the invention. Preferably, the at least 5 genes are selected from one of the following groups or a combination thereof:
a) RPIB9, CXCL2, AL137761, TFPI2, THC2051204, TNF, ABCB1, PURG, ADAMTS5, MCTP1, SPTLC2L, OAS3, MCTP1, GAS1, BIRC3, BQ186674, MAL, UBXD3, WNT2B, GALNT14, TM4SF1, ZAR1, A_23_P10091, GLT8D2, RXFP1, CGNL1, AK094972, LRCH2, BM930757, ATP8A1, SOX9, SLC39A8, TMEM47, SLC10A4, SLC1A3, EDG7, ITGA2, PLCXD3, BF514799, SLC16A12, THC2208430, THC2182743, C4orf18, ANKRD38, CALCRL, and LOC152573, preferably RPIB9, CXCL2, TFPI2, TNF, ABCB1, ADAMTS5, PURG, OAS3, GAS1, BIRC3, MAL, GALNT14, TM4SF1, RXFP1, ATP8A1, SOX9, SLC39A8, EDG7, ITGA2, SLC1A3, CALCRL and LOC152573;
b) RPIB9, TFPI2, ABCB1, BIRC3, WNT2B, SFRP1, FSTL1, AHR, CDKN1C, ABCB2, CYR61, WNT5A, ABCC3, JAG1, STAT1, WNT7B, CASP8, LZTS1, FZD8, GALNT14, RXFP1 and LOC152573;
c) ABCC3, CD55, COL16A1, DHRS3, FSTL1, GLS, HDL, HIVE1, LAMA2, LAMB3, LIPG, LITAF, MAL, MFHAS1, NFKBIZ, NRP1, NRP2, OAS3, OLR1, PSCDBP, RFTN1, SCARAB1, SEMA3B, SEMA3C, SFRP1, SLC1A3, ST6GAL1, TLR3, TM4SF1 and TNF;
d) ADAMTS1, ADRA2C, AKAP12, CDKN1C, CYR61, FBN1, GAS1, GPC3, IGF2, IGFBP3, JAG1, MGST1, NTN4, PDE1A, PDE4B, PDE4D, PDE4DIP, PDGFB, PHLDA1, PIM1, PPP2R2C, RGS16, SCD, SLC1A1, SMPDL3A, TFPI2 and VCAN;
e) ABCB1, AHR, AHRR, AMPH, BIRC3, CXCL2, CYP1A1, IL1R1, NQO1, PLAT, PLXNA2, SLC16A10, SLC3A1, SLC7A8, SLPI, TAP1, UGT8, UGT2B4, UGT2B7, UGT2B10, UGT2B11 and UGT2B28;
f) AQP1, ARHGDIB, BAMBI, CREB5, CXCR4, EPAS1, FGF2, FGFBP1, GRB10, IL15, MT2A, NUPR1, PDK1, PROS1, PTPN3, RPS6KA2, TFDP2, WNT2B, WNT5A and WNT7B;
g) AGT, ATP8A2, BDNF, EDG6, GAL, GATA2, ITGA2, LRP11, LZTS1, MYB, NCALD, PNOC, PTGES, SRGAP3, TAC3 and TTN;
h) AFF1, ASGR1, BLVRA, CASP8, CD40, KCNH2, NRG1, NRL, PHEX, PLAC8, SMAD7, SMAD9, SOX9, SPG20 and STAT1;
i) TNF, ABCB1, CYP1A1, AHRR, AHR, PP2R2C, ABCC3, NQO1, PIK3C3, UGT2B7, UGT2B11, UGT2B28, UGT2B4, UGT2B10, CHST7, MGST1 and UGT8; and,
j) Wnt2B, Wnt5A, Wnt7B, SFRP1, FSTL1, Jag1, Cyr61, LOC152573, FZD8 and FOXL2.

The present invention also relates to the use of a DNA chip or a kit of the invention for preparing a kit for predicting or monitoring whether a patient affected by a cancer is responsive to a treatment with a molecule of the taxoid family. Preferably, the cancer is selected from the group consisting of the breast cancer, the lung cancer, the prostate cancer, the gastric cancer and the head and neck cancer. More preferably the cancer is the prostate cancer. In a preferred embodiment, the molecule of the taxoid family is selected from the group consisting of docetaxel, larotaxel, XRP6258, BMS-184476, BMS-188797, BMS-275183, ortataxel, RPR 109881A, RPR 116258, NBT-287, PG-paclitaxel, ABRAXANE®, Tesetaxel, IDN 5390, Taxoprexin, DHA-paclitaxel, and MAC-321. More preferably, the molecule of the taxoid family is docetaxel.

The present invention further concerns methods for screening or identifying a compound suitable for improving the treatment of a cancer with a molecule of the taxoid family or for reducing the resistance development during the treatment of a cancer with a molecule of the taxoid family. In a first embodiment, the method comprises: 1) providing a cell-line with at least 5, 6, 7, 8, 9, 10, 15, 20, 25, 30, 40, 50, 60, 70, 80, 90, 100, 150, 200 or 300 genes over-expressed and/or under-expressed respectively selected from the group of over-expressed genes of Table 1 and under-expressed genes of Table 2; 2) contacting said cell-line with a test compound; 3) determining the expression level of said at least 5, 6, 7, 8, 9, 10, 15, 20, 25, 30, 40, 50, 60, 70, 80, 90, 100, 150, 200 or 300 genes; and, 4) selecting the compound which decreases the expression level of over-expressed genes and increases the expression level of under-expressed genes. In a second embodiment, the method comprises: 1) providing a cell-line sensitive to the molecule of the taxoid family; 2) contacting said cell-line with a test compound and the molecule of the taxoid family; 3) determining the expression level of said at least 5, 6, 7, 8, 9, 10, 15, 20, 25, 30, 40, 50, 60, 70, 80, 90, 100, 150, 200 or 300 genes selected from the genes listed in Tables 1 and 2; and, 4) selecting the compound which inhibits the appearance of an over-expression and/or an under-expression of at least 5, 6, 7, 8, 9, 10, 15, 20, 25, 30, 40, 50, 60, 70, 80, 90, 100, 150, 200 or 300 genes respectively selected from the group of genes of Table 1 and genes of Table 2. In a third embodiment, the method comprises: 1) providing a cell-line with at least on gene over-expressed and/or under-expressed respectively selected from the group consisting of RPIB9, CXCL2, AL137761, TFPI2, THC2051204, TNF, ABCB1, PURG, ADAMTS5, MCTP1, SPTLC2L, OAS3, MCTP1, GAS1, BIRC3, BQ186674, MAL, UBXD3, and WNT2B for the over-expressed genes, and GALNT14, TM4SF1, ZAR1, A_23_P10091, GLT8D2, RXFP1, CGNL1, AK094972, LRCH2, BM930757, ATP8A1, SOX9, SLC39A8, TMEM47, SLC10A4, SLC1A3, EDG7, ITGA2, PLCXD3, BF514799, SLC16A12, THC2208430, THC2182743, C4orf18, ANKRD38, CALCRL, and LOC152573 for the under-expressed genes; 2) contacting said cell-line with a test compound; 3) determining the expression level of said at least one gene; and, 4) selecting the compound which decreases the expression level of over-expressed genes and increases the expression level of under-expressed genes.

Preferably, the cell-line is a cancer cell-line. In particular, the cancer cell-line is specific of the targeted cancer. For instance, if the prostate cancer is to be treated, then the cell-line is a prostate cancer cell-line.

In a preferred embodiment, the molecule of the taxoid family is selected from the group consisting of docetaxel, larotaxel, XRP6258, BMS-184476, BMS-188797, BMS-275183, ortataxel, RPR 109881A, RPR 116258, NBT-287, PG-paclitaxel, ABRAXANE®, Tesetaxel, IDN 5390, Taxoprexin, DHA-paclitaxel, and MAC-321. More preferably, the molecule of the taxoid family is docetaxel. Preferably, the cancer is selected from the group consisting of the breast cancer, the lung cancer, the prostate cancer, the gastric cancer and the head and neck cancer. More preferably the cancer is the prostate cancer.

The example illustrates the invention without limiting its scope.

### EXAMPLES

### Methods

### Cell culture and selection of docetaxel-resistant clones

The human androgen-independent prostate carcinoma cell line PC3 was obtained from American Type Culture Collection (Rockville, MD, USA) and was maintained in DMEM medium containing 10% heat-inactivated fetal bovine serum (FBS) with 100U/ml penicillin, 100 mg/ml streptomycin. The human androgeno-dependent prostate carcinoma cell line LNCaP was maintained in RPMI medium complemented with 10% FBS and antibiotics. The human androgen-independent IGR-CaP1 cell line recently obtained for a localized prostate cancer was maintained in RPMI medium complemented with 10% FBS and antibiotics. Docetaxel-resistant clones were selected by culturing the cells in docetaxel in a dose-escalation manner. Initial culture was done in 0.5nM docetaxel. Cellular clones surviving in the presence of 0.5nM docetaxel were maintained in culture during four passages, and then the concentration of docetaxel in the medium was increased to 2.5nM and subsequently to 12nM, 25nM, 50nM, 100nM and 200nM. The same selection methodology was followed with each increase in docetaxel concentration. Once cells were freely dividing in each dose of docetaxel mediums, they were considered as resistant and labelled IGR-CaP1-R, LNCaP-R and PC3-R. IGR-CaP1-R clones were obtained surviving in medium containing respectively 2.5nM, 5nM, 12nM, 25nM, 50nM, 100nM and 200nM docetaxel. LNCaP-R clones survived in medium containing 0.5nM, 2.5nM and 5nM docetaxel. PC3-R clones survived in medium containing 0.5nM and 2.5nM docetaxel. All the cell cultures were maintained at 70% confluency and medium was changed every 48 h.

### Cell Cycle analysis

Effects of treatments on the stages of the cell cycle were determined using the PI staining technique. Briefly, parental and docetaxel-resistant IGR-CaP1 cells were grown in flasks at a density of 4x10⁶ cells. After allowing for overnight attachment the cells were treated or not with 12nM docetaxel. Cells were incubated for 48 hr then collected by trypsinization, making sure to include the floating cells. After washing in PBS the cells were fixed and permeabilized using Fix&Perm kit (InVitrogen) according to the manufacturer protocol. Cells were treated with 20µg DNAse-free RNAse for 30 min and stained with 100µg propidium iodide (PI) for 30 min. Then percentage of cells in G1, S, G2, M, and subG1 phases were analyzed with FACS Calibur cytometer (Becton Dickinson).

### Total RNA Preparation and Reverse Transcription

Total RNA from parental and docetaxel-resistant IGR-CaP1 cells was isolated using TriReagent (Sigma-Aldrich) and purified with RNeasy Micro Kit (Qiagen) according to manufacturer's protocols. Quality of RNA preparation, based on the RNA Integrity Number (RIN), was assessed using the Agilent RNA 6000 Nano Kit as developed on the Agilent 2100 Bioanalyzer device (Agilent Technologies, Palo Alto, CA). All specimens included in this study displayed a RIN of 10. RNA samples were frozen in nuclease-free water (Qiagen).

### Oligo Microarray Technology

Parental and resistant-cell line total RNAs were directly compared by using Agilent oligonucleotide dual-color technology, running dye-swap and duplicate experiments. Total RNA from the parental IGR-CaP1 cell line without treatment was used as the RNA reference. Total RNA from IGR-CaP1 cells resistant to treatment with 5nM, 12nM, 25nM and 50nM of docetaxel respectively, were used as samples. Probe synthesis and labeling were performed by Agilent's Low Fluorescent Low input Linear Amplification Kit. Hybridization was performed on the Agilent 4x44K Human 1A (G4112F) long (60-bp) oligonucleotide microarrays (Agilent Technologies) by using reagents and protocols provided by the manufacturer. Feature extraction software provided by Agilent (Version A.9.5.3.1) was used to quantify the intensity of fluorescent images and to normalize results using the linear and lowess subtraction method. Primary analysis was performed by using Resolver software (version 7.1) (Rosetta Laboratories, Milan) to identify genes differentially expressed between parental and resistant cell lines with a fold change > 2 and *P* value < 10⁻¹⁰. Using this procedure for each of the 4 combined experiments, a list of 378 genes was extracted and was considered as a signature of gene potentially implicated in resistance to docetaxel. These genes were sorted out by the mean of the fold change observed respectively for the 4 doses of resistance towards docetaxel.

### TaqMan Real-Time Quantitative Reverse Transcription-PCR Analysis.

Real-time quantitative RT-PCR was performed using the ABI Prism 7900 Sequence Detection System (Perkin-Elmer Applied Biosystems). The same procedure was applied from the total RNA used in the microarray analysis and for independent RNA samples. One µg of total RNA was reversed transcribed using the GeneAmp RNA PCR Kit according to the manufacturer's recommendations (Applied Biosystems).

Quantitative real-time PCR was performed in a final volume of 25 µl according to the manufacturer's recommendations (Applied Biosystems). PCR primers and probe for the selected target genes were designed by Applied Biosystems and used according to the manufacturer's recommendations. The amount of sample RNA was normalized by the amplification of an endogenous control (18S). The relative quantification of the transcripts was derived by using the standard curve method (Applied Biosystems User Bulletin 2, ABI PRISM 7700 Sequence Detection System). The following Taqman probes were used : RPIB9 Hs00379227_ml and Hs00289927_m1; ABCB1 Hs00184491_m1; ABCB2 Hs00388682_ml; ABCC3 Hs00358656_ml: BIRC3 Hs00154109_ml; TFPI2 Hs00197918_ml; STAT1 Hs00234829_ml; CLU Hs00156548_ml, AHR Hs00907314_ml; CDKN1C Hs00175938_m1; GALNT14 Hs00226180_m1; CASP8 Hs01018151_ml; LZTS1 Hs00232762_ml; LOC152573 Hs01380806_ml; WN2B Hs00244632_ml and Hs00257131_ml; WNT5A Hs00998537_ml; WNT7B Hs00536497_ml; SFRP1 Hs00610060_ml; FSTL1 Hs00200053_ml; JAG1 Hs01070032_ml; FDZ8 Hs00259040_s1.

### Western blot analysis

Parental and resistant cellular clones were cultured in 175cm2 flask in the presence of the appropriate concentration of docetaxel. Cells were lysed in RIPA buffer to prepare whole cell extracts and denatured in NuPage LDS sample buffer (Invitrogen). Protein concentration of the soluble extracts was determined by using the MicroBCA protein assay (Pierce).

Proteins from 50µg of whole cell extracts were resolved by electrophoresis on NuPage 4-12% Bis-Tris gels (Invitrogen) and immunoblots were developed using the enhanced chemoluminescence-based detection kit (Pierce). The following antibodies were used: anti-ABCB1 (Mdr-1 D-11) and anti-LZTS1 (FEZ1 C-20) from Santa-Cruz Biotechnology Inc. The equal loading of protein sample was verified with a β-actin-specific antibody (Sigma).

### RESULTS

**Generation of acquired resistance to Docetaxel *in vitro.*** Prostate cancer IGR-CaP1 cells were used to generate successive docetaxel-resistant cell lines. The addition of docetaxel induced a selection process, whereby a large majority of cells initially underwent cell death until the ability to proliferate was regained. The inventors obtained IGR-CaP1 resistant (IGR-CaP1-R) clones which survived in medium containing respectively 5nM, 12nM, 25nM, 50nM of docetaxel. Cell cycle analysis was done to show acquired resistance to drug. The resistant cell lines showed cell cycle similar to the parental IGR-CaP1 cells, suggesting that acquired resistance had been gained (not shown).

**Genome-wide analysis of docetaxel-resistant lines using microarray**. Human genome-wide analysis of gene expression changes was realized in order to stringently identify human genes that might represent the molecular signature of resistance or sensitivity to docetaxel in prostate cancer. Untreated IGR-CaP1 parental cell lines were used as baseline. Hierarchical clustering of combined experiments using a 2-fold change criteria and a *P* value of <10⁻¹⁰ revealed a total of 378 genes that were up or down-regulated by >2-fold in each of the resistant cell lines. 191 genes were over-expressed (Table 1) and 187 were down-regulated (Table 2) in docetaxel-resistant cells. These genes were sorted out by the mean of the fold change observed respectively for the 4 doses of resistance towards docetaxel (Table 3 and Table 4).. Functional analysis of the resistant cell lines was performed using Ingenuity® Pathways Analysis (IPA). Highly significant functions and canonical pathways were found for resistant cell lines as organ development, cancer, cellular growth and proliferation, cellular movement, cell-to-cell signalling and interaction, or cell death.

### Target verification by real-time RT-PCR and western blot

To verify the alterations of gene expression at the mRNA level, which appeared on the microarray, the inventors chose representative genes with varying expression profiles for real-time Taqman RT-PCR and Western Blot analysis. The inventors measured gene expression levels in a panel of 22 genes.

The inventors first measured the expression of the Top gene of the signature, RPIP9/RPIB9/RUNDC3B, encoding Rap2-binding protein 9. Two sets of probes were chosen to measure gene expression of RPIB9 as multiple splice variants were transcribed (Fig 1A). The two probes showed a high level in gene expression in docetaxel-resistant cells in a dose dependent manner (Fig1B). Over expression was more pronounced (more than 1000 fold) with the 3' probe set, suggesting that long variants containing RUN domain were more expressed. The same results were obtained on an independent set of total RNAs (not shown). The function of RPIP9 protein is not known but RPIP9 gene was shown to be overexpressed in breast carcinoma and correlated with a poor prognosis.

A key mechanism underlying multidrug resistance relates to the overexpression of the ATP-dependent transporter family known as the ATP-binding cassette (ABC) family. One of the most described members of these drug efflux pumps was the P-glycoprotein (P-gp) encoded by the MDR-1 gene. This gene had been frequently found overexpressed in drug-resistant phenotype. The gene ABCB1/MDR1 is one of the most over-expressed genes of the signature. The same alterations of gene expression were observed by real-time RT-PCR analysis, although the fold change in the expression level was much higher (Fig 2A). The same results were obtained on an independent set of total RNAs (not shown). Western blot analysis showed that expression of the MDR1 gene product was increased in a dose-dependent manner in docetaxel-resistant cells (Fig 2B). Two other genes encoding members of the ATP-binding cassette family, ABCB2 and ABCC3 were confirmed to be overexpressed in resistant cells, although to a lesser level compared to the ABC gene (Fig 2C). Interestingly, ABCC3 was recently identified as a mediator of taxane resistance in HER2-amplified breast cancer.

The genes BIRC3 and TFPI2 were found in the Top 15 of over-expressed genes of the signature with a fold-change expression of 10.4 and 21.8 respectively in the resistant cells. BIRC3 encoding baculoviral IAP repeat-containing 3 belongs to a family of proteins that inhibits apoptosis (IAP family). Interestingly, it had been suggested that IAP proteins may have an important contribution to the resistance to the apoptotic effect of cisplatin in prostate cancer. The TFPI2 gene, encoding tissue factor pathway inhibitor 2, is a potent inhibitor of matrix-metalloproteinase. This protein was shown to be most prominently up-regulated in MYCN-amplified neuroblastomas. RT-PCR analysis confirmed that BIRC3 and TFPI2 genes were overexpressed in taxane-resistant cells up to 36 fold and 64 fold respectively (Fig 3).

As the genes STATE1 and clusterin were showed overexpressed in DU145-DR and PC3-DR docetaxel-resistant cells in the study of Patterson et al., (2006), the inventors verified the expression level of these two genes by RT-PCR in the IGR-CaP1-R model. STAT1 was also found overexpressed in the present signature with a fold change of 2.47 but Clusterin was not retained in the present microarray analysis. As shown in Fig 4A, the genes STAT1 and Clusterin were up-regulated in IGR-CaP1-R resistant cells, although to a modest extent. CDKN1C was another gene that had been shown overexpressed in DU145-DR and PC3-DR docetaxel-resistant cells. In the present microarray analysis, the inventors found that CDKN1C gene was also overexpressed with a 5.38 fold change in resistant cells. This result was confirmed by the RT-PCR experiment (Fig 4B). AHR is a ligand-activated transcription factor that mediates a pleiotropic response to environmental contaminants and that has recently been shown to be implicated in the development of cancers from different anatomical origins. Moreover, AHR had been identified as a putative Wnt/β-Catenin pathway target gene in prostate cancer cells. The AHR gene showed a 5.40 fold overexpression in resistant cells in the present microarray analysis. The inventors confirmed this result and showed a high dose-dependent overexpression in taxane-resistant cells by the RT-PCR approach (Fig 4B). The high increase in AHR gene expression was confirmed on an independent set of total RNA (not shown).

GALNT14 belongs to a large subfamily of glycosyltransferases residing in the Golgi apparatus. GALNT enzymes catalyze the first step in the O-glycosylation of mammalian proteins by transferring N-acetyl-D-galactosamine (GalNAc) to peptide substrates. The GALNT14 gene was one of the top down-regulated genes in the present signature with a fold-change expression of -10.26 in the resistant cells. The dose-dependent down-regulation of the expression of GALNT14 in resistant cells was confirmed by the RT-PCR analysis (Fig 5). The high decrease in GALNT14 gene expression was confirmed on an independent set of total RNA (not shown). The caspase 8 gene encodes a member of the cysteine-aspartic acid protease (caspase) family. Sequential activation of caspases plays a central role in the execution-phase of cell apoptosis. This gene was founded moderately under-expressed in the RT-PCR analysis (Fig 5).

LZTS1 encoding leucine zipper, putative tumor suppressor 1 was shown under-expressed in the present signature. The under-expression of this gene was confirmed by RT-PCR analysis (Fig 6A) and western blot analysis (Fig 6B) in docetaxel-resistant cells. The same results were obtained on an independent set of total RNAs (not shown). LZTS1 was of particular interest since it has been described as a tumor suppressor gene. The FEZ1/LZTS1 (LZTS1) protein was shown to be frequently downregulated in esophageal, breast, and prostate cancers. LZTS1 is expressed in normal tissues, and its introduction in cancer cells inhibits cell growth and suppresses tumorigenicity. Absence or low expression of LZTS 1 was correlated to high tumor grading on lung tumors suggesting that it may serve as a novel prognostic indicator.

The gene LOC152573 encodes the hypothetical protein BC012029 also named hSHISA3. The function of hSHISA3 is not known but by analogy with the mouse homologs, it is supposed to play an essential role in the maturation of presomitic mesoderm cells by individual attenuation of both FGF and WNT signalling. LOC152573/hSHISA3 corresponded to the most under-regulated gene in resistant cells with a fold change of -159.40 in the microarray analysis. The inventors confirmed the high decrease of its expression on independent set of total RNAs of resistant IGR-CaP1-R cells (Fig7A) as well as in extracts obtained from LNCaP-R and PC3-R docetaxel-resistant cells (Fig 7B).

Finally, the inventors checked for the confirmation of the genes implicated in the WNT pathway that were recovered in the microarray analysis. WNT family members function in a variety of developmental processes including regulation of cell growth and differentiation and are characterized by a WNT-core domain. Additionally, WNT signaling has emerged as an important pathway that underlies the initial notion of prostate cancer. Both human cancers and mouse models have confirmed that mutations or altered expression of components of this pathway are associated with prostate tumors. The WNT2B encoding a member of the WNT family of highly conserved, secreted signalling factors, was shown as one of the most over-expressed gene in the signature with a fold change of 9.42 in resistant cells. Two sets of probes were chosen to measure gene expression of WNT2B as this gene produces two alternative transcript variants (Fig 8A). The two probes showed a high level in gene expression in docetaxel-resistant cells in a dose dependent manner (Fig 8B). Others members of the WNT gene family, WNT5A and WNT7B genes, were also showed to be overexpressed in drug-resistant cells, although to a lesser extent (Fig 8C). The gene SFRP1 (Secreted frizzled-related protein 1) acting as soluble modulator of WNT signalling, FSTL1 encoding a protein with similarity to follistatin, and JAG1 encoding the ligand for the receptor Notch 1 were also shown to be up-regulated in the drug-resistant cells, although to different extent (Fig 8D). On the contrary, the gene FZD8, encoding a member of the frizzled gene family, showed a high decrease of its expression in drug-resistant cells (Fig 8E).

Overall, the results of real-time RT-PCR for these selected genes were in direct agreement with the microarray data. The same alternations of gene expression were observed by real-time RT-PCR analysis, although the fold change in the expression level was not exactly same between these two different analytical methods. Western Blot analyses were also in direct agreement with the microarray data. These results support the findings obtained from the present microarray analysis.

**Table 1 : List of the over-expressed genes (at least two-fold) in the docetaxel resistant cell-lines.**

| **Primary Sequence Name** | **Sequence Name(s)** | **Sequence Description** | **Accession #** |
|---|---|---|---|
| RPIB9 | RPIB9,RPIP9,FLJ30671,MG C26655 | Rap2-binding protein 9 | NM_138290 |
| CXCL2 | CXCL2,GRO2,GROb,MIP2, MIP2A,SCYB2,MGSA-b,MIP-2a,CINC-2a,MGSA beta | GR02 oncogene | NM_002089 |
| AL137761 | AL137761 | Homo sapiens mRNA; cDNA DKFZp586L2424 (from clone DKFZp586L2424), [AL137761] | AL137761 |
| TFP12 | TFP12,PP5,TFP1-2,FLJ21164 | tissue factor pathway inhibitor 2 | NM_006528 |
| THC2051204 | THC2051204 | Q300_MOUSE (Q02722) Protein Q300, partial (17%) [THC2051204] | |
| TNF | TNF,DIF,TNFA,TNFSF2,TNF -alpha | tumor necrosis factor (TNF superfamily, member 2) | NM_000594 |
| ABCB1 | ABCB1,CLCS,MDR1,P-gp,PGY1,ABC20,CD243,GP 170 | ATP-binding cassette, sub-family B (MDR/TAP), member 1 | NM_000927 |
| PURG | PURG,PURG-A,PURG-B,MGC119274 | purine-rich element binding protein G | M_013357 |
| ADAMTS5 | ADAMTS5,ADMP-2,ADAMTS11,FLJ36738 | a disintegrin-like and metalloprotease (reprolysin type) with thrombospondin type 1 motif, 5 (aggrecanase-2) | NM_007038 |
| MCTP1 | MCTP1,FLJ22344 | Homo sapiens cDNA FLJ34011 fis, clone FCBBF2001868, weakly similar to RABPHILIN-3A, [AK091330] | AK091330 |
| SPTLC2L | | Homo sapiens cDNA FLJ90790 fis, clone THYR01001529, weakly similar to SERINE PALMITOYL TRANSFERASE 2 (EC 2,3,1,50), [AK075271] | AK075271 |
| OAS3 | OAS3,p100,MGC133260 | 2'-5'-oligoadenylate synthetase 3 (100 kD) | NM_006187 |
| MCTP1 | MCTP1,FLJ22344 | hypothetical protein FLJ22344 | NM_024717 |
| GAS1 | GAS1 | growth arrest-specific 1 | NM_002048 |
| BIRC3 | BIRC3,AIP1,AP12,MIHC,CIA P2,HAIP1,HIAP1,MALT2,RN F49 | baculoviral IAP repeat-containing 3 | M_001165 |
| BQ186674 | BQ186674 | UI-E-EJ1-ajr-f-10-0-UI,rl UI-E-EJ1 Homo sapiens cDNA clone UI-E-EJ1-ajr-f-10-0-UI 5', mRNA sequence [BQ186674] | BQ186674 |
| MAL | MAL | mal, T-cell differentiation protein | NM_002371 |
| UBXD3 | UBXD3,FLJ25429 | Homo sapiens UBX domain containing 3 (UBXD3), mRNA [NM_152376] | NM_152376 |
| WNT2B | WNT2B,WNT13,XWNT2 | wingless-type MMTV integration site family, member 2B | NM_024494 |
| BM716045 | BM716045 | UI-E-EJO-aht-1-14-0-UI,r1 UL-E-EJO Homo sapiens cDNA clone UI-E-EJO-aht-1-14-0-UI 5', mRNA sequence [BM716045] | BM716045 |
| SFRP1 | SFRP1,FRP,FRP1,FrzA,FRP -1,SARP2 | secreted frizzled-related protein 1 | NM_003012 |
| PLEKHH2 | PLEKHH2,KIAA2028,PLEKH H1L | Homo sapiens pleckstrin homology domain containing, family H (with MyTH4 domain) member 2 (PLEKHH2), mRNA [NM_172069] | NM_172069 |
| GNG11 | GNG11,GNGT11 | guanine nucleotide binding protein 11 | NM_004126 |
| CDH16 | CDH16 | cadherin 16, KSP-cadherin | NM_004062 |
| AKR1C1 | AKR1C1,C9,DD1,DDH,DDH 1,H-37,MBAB,HAKRC,MGC8954 ,2-ALPHA-HSD,20-ALPHA-HSD | aldo-keto reductase family 1, member C1 (dihydrodiol dehydrogenase 1; 20-alpha (3-alpha)-hydroxysteroid dehydrogenase) | NM_001353 |
| MGC42367 | MGC42367 | similar to 2010300C02Rik protein | NM_207362 |
| SFRP1 | SFRP1,FRP,FRP1,FrzA,FRP -1,SARP2 | secreted frizzled-related protein 1 | NM_003012 |
| AQP1 | AQP1,CO,CHIP28,AQP-CHIP,MGC26324 | Homo sapiens aquaporin 1 (channel-forming integral protein, 28kDa) (AQP1), transcript variant 1, mRNA [NM_198098] | NM_198098 |
| RAFTLIN | RAFTLIN,MIG2,PIG9,PIB10, KIAA0084,MGC141678 | raft-linking protein | NM_015150 |
| FAM111A | FAM111A,FLJ22794,KIAA18 95,DKFZp686A06175 | hypothetical protein FLJ22794 | NM_022074 |
| FAM111A | FAM111A,FLJ22794,KIAA18 95,DKFZp686A06175 | hypothetical protein FLJ22794 | NM_022074 |
| ADAMTS1 | ADAMTS1,C3-C5,METH1,KIAA1346 | a disintegrin-like and metalloprotease (reprolysin type) with thrombospondin type 1 motif, 1 | NM_006988 |
| FHOD3 | FHOD3,FHOS2,Formactin2 | hypothetical protein FLJ22297 | NM_025135 |
| DUSP23 | DUSP23,VHZ,LDP-3,DUSP25,FLJ20442,RP11-190A12,1 | hypothetical protein FLJ20442 | NM_017823 |
| ITGB8 | ITGB8 | Homo sapiens, clone IMAGE:4794726, mRNA, [BC042028] | BC042028 |
| ADAMTS1 | ADAMTS1,C3-CS,METH1,KIAA1346 | a disintegrin-like and metalloprotease (reprolysin type) with thrombospondin type 1 motif, 1 | NM_006988 |
| THC2134488 | THC2134488 | Unknown | |
| IL15 | IL15,IL-15,MGC9721 | Homo sapiens interleukin 15 (IL15), transcript variant 1, mRNA [NM_172174] | NM_172174 |
| PLEKHH2 | PLEKHH2,KIAA2028,PLEKH H1L | Homo sapiens pleckstrin homology domain containing, family H (with MyTH4 domain) member 2 (PLEKHH2), mRNA [NM_172069] | NM_172069 |
| AKR1C1 | AKR1C1,C9,DD1,DDH,DDH 1,H-37,MBAB,HAKRC,MGC8954 ,2-ALPHA-HSD,20-ALPHA-HSD | aldo-keto reductase family 1, member C1 (dihydrodiol dehydrogenase 1; 20-alpha (3-alpha)-hydroxysteroid dehydrogenase) | NM_001353 |
| IGFBP3 | IGFBP3,IBP3,BP-53 | insulin-like growth factor binding protein 3 | NM_000598 |
| CYP1A1 | CYP1A1,AHH,AHRR,CP11, CYP1,P1-450,P450-C, P450DX | cytochrome P450, subfamily I (aromatic compound-inducible), polypeptide 1 | NM_000499 |
| PHLDA1 | PHLDA1,PHRIP,TDAG51,DT 1P1B11,MGC131738 | pleckstrin homology-like domain, family A, member 1 | AF220656 |
| TLR3 | TLR3,CD283 | toll-like receptor 3 | NM_003265 |
| GPC3 | GPC3,SGB,DGSX,SDYS,SG BS,SGBS1 | glypican 3 | NM_004484 |
| AHRR | AHRR,AHH,AHHR,KIAA123 4 | Homo sapiens aryl-hydrocarbon receptor repressor (AHRR), mRNA [NM_020731] | NM_020731 |
| CACNG6 | CACNG6 | Homo sapiens calcium channel, voltage-dependent, gamma subunit 6 (CACNG6), transcript variant 1, mRNA [NM_145814] | NM_145814 |
| AQP1 | AQP1,CO,CHIP28,AQP-CHIP,MGC26324 | aquaporin 1 (channel-forming integral protein, 28kD) | NM_000385 |
| AKR1C3 | AKR1C3,DD3,HAKRB,HAKR e,HA1753,HSD17B5,hIuPGF S,KIAA0119 | aldo-keto reductase family 1, member C3 (3-alpha hydroxysteroid dehydrogenase, type II) | NM_003739 |
| FSTL1 | FSTL1,FRP,FSL1, Follistatin-like | follistatin-like 1 | NM_007085 |
| AHR | AHR | aryl hydrocarbon receptor | NM_001621 |
| C1orf88 | C1orf88,FLJ23853,MGC126 550,RP5-1125M8,4 | Homo sapiens hypothetical protein LOC128344 (LOC128344), mRNA [NM_181643] | NM_181643 |
| CDKN1C | CDKN1C,BWS,WBS,p57,BW CR,KIP2 | cyclin-dependent kinase inhibitor 1C (p57, Kip2) | NM_000076 |
| A_32_P32463 | A_32_P32463 | Unknown | |
| PCDH9 | PCDH9 | protocadherin 9 | NM_020403 |
| NTN4 | NTN4,PR03091,FLJ23180 | netrin 4 | M_021229 |
| MID1 | MID1,OS,FXY,OSX,OGS1,X PRF,BBBG1,GBBB1,RNF59, ZNFXY,TRIM18 | midline 1 (Opitz/BBB syndrome) | NM_033290 |
| CSPG2 | CSPG2,VERSICAN,DKFZp6 86K06110 | chondroitin sulfate proteoglycan 2 (versican) | NM_004385 |
| AL133090 | AL133090 | Homo sapiens mRNA; cDNA DKFZp434E0528 (from clone DKFZp434E0528), [AL133090] | AL133090 |
| DDC | DDC,AADC | dopa decarboxylase (aromatic L-amino acid decarboxylase) | NM_000790 |
| AKR1C1 | AKR1C1,C9,DD1,DDH,DDH 1,H-37,MBAB,HAKRC,MGC8954 ,2-ALPHA-HSD,20-ALPHA-HSD | aldo-keto reductase family 1, member C1 (dihydrodiol dehydrogenase 1; 20-alpha (3-alpha)-hydroxysteroid dehydrogenase) | NM_001353 |
| PHLDA1 | PHLDA1,PHRIP,TDAG51,DT 1P1B11,MGC131738 | pleckstrin homology-like domain, family A, member 1 | NM_007350 |
| KCNH2 | KCNH2,ERG1,HERG,LQT2, SQT1,HERG1,Kv11,1 | potassium voltage-gated channel, subfamily H (eag-related), member2 | NM_000238 |
| ITGB8 | ITGB8 | integrin, beta 8 | NM_002214 |
| ST6GAL1 | ST6GAL1,CD75,SIAT1,ST6 Gall, MGC48859,ST6Gal I | Homo sapiens sialyltransferase 1 (beta-galactoside alpha-2,6-sialyltransferase) (SIAT1), transcript variant 1, mRNA [NM_173216] | NM_173216 |
| TMEFF1 | TMEFF1,H7365,C9orf2 | transmembrane protein with EGF-like and two follistatin-like domains 1 | NM_003692 |
| FBXL16 | FBXL16,FbI16,C16orf22,FLJ 33735,MGC33974,c380A1,1 | Homo sapiens F-box and leucine-rich repeat protein 16 (FBXL16), mRNA [NM_153350] | NM_153350 |
| ATP8A2 | ATP8A2,IB,ATP,ML-1,ATPIB,DKFZP434B1913 | ATPase, aminophospholipid transporter-like, Class I, type 8A, member 2 | AL390129 |
| CART1 | CART1 | cartilage paired-class homeoprotein 1 | NM_006982 |
| C9orf150 | C9orf150,bA3L8,2,FLJ38505 ,FLJ90271,HYST0841,MGC4 6502 | Homo sapiens chromosome 9 open reading frame 150 (C9orf150), mRNA [M_203403] | NM_203403 |
| PHLDA1 | PHLDA1,PHRIP,TDAG51,DT 1P1B11,MGC131738 | Homo sapiens cDNA clone IMAGE:5531727, partial cds, [BC037430] | BC037430 |
| HDAC9 | HDAC9,HD7,HDAC,HDRP,M ITR,HDAC7,HDAC7B,HDAC 9B,HDAC9FL,KIAA0744,DK FZp779K1053 | histone deacetylase 7B | NM_014707 |
| GLS | GLS,GLS1,FLJ10358,KIAA0 838,DKFZp686O15119 | glutaminase | NM_014905 |
| GATS | GATS,DKFZp686B07267 | opposite strand transcription unit to STAG3 | BC065200 |
| CNTNAP3 | CNTNAP3,CASPR3,CNTNA P3A,RP11-290L7,1,RP11-138L21,1 | cell recognition molecule CASPR3 | NM_033655 |
| PDE4B | PDE4B,DPDE4,PDEIVB,MG C126529,DKFZp686F2182 | phosphodiesterase 4B, cAMP-specific (dunce (Drosophila)-homolog phosphodiesterase E4) | L12686 |
| DKFZp586I14 20 | DKFZp586I1420 | Homo sapiens hypothetical protein DKFZp586I1420 (DKFZp586I1420) on chromosome 7 [NRL_002186] | NRL_002186 |
| ZNRF2 | ZNRF2,RNF202 | Homo sapiens zinc and ring finger 2 (ZNRF2), mRNA [NM_147128] | NM_147128 |
| FGF2 | FGF2,BFGF,FGFB,HBGH-2 | fibroblast growth factor 2 (basic) | NM_002006 |
| SP5 | SP5 | Homo sapiens Sp5 transcription factor (SP5), mRNA [M_001003845] | NM_001003 845 |
| LAMA2 | LAMA2,LAMM | laminin, alpha 2 (merosin, congenital muscular dystrophy) | NM_000426 |
| THC2056328 | THC2056328 | Unknown | |
| KIAA1666 | KIAA1666,DKFZp434H0735 | KIAA1666 protein | AL117509 |
| A_23_P10395 1 | A_23_P103951 | Unknown | |
| IL1R1 | IL1R1,P80,IL1R,IL1RA,CD12 1A,D2S1473,IL-1R-alpha | interleukin 1 receptor, type I | NM_000877 |
| CD55 | CD55,CR,TC,DAF | decay accelerating factor for complement (CD55, Cromer blood group system) | NM_000574 |
| MANEAL | MANEAL,FLJ31434,MGC78 681,RP11-109P14,3 | Homo sapiens hypothetical protein FLJ31434 (FLJ31434), mRNA [NM_152496] | NM_152496 |
| AK026140 | AK026140 | Homo sapiens cDNA: FLJ22487 fis, clone HRC10931, [AK026140] | AK026140 |
| FXYD2 | FXYD2,HOMG2,ATP1G1,M GC12372 | FXYD domain-containing ion transport regulator 2 | NM_021603 |
| CD40 | CD40,p50,Bp50,CDW40,MG C9013,TNFRSF5 | tumor necrosis factor receptor superfamily, member 5 | NM_001250 |
| KIAA1505 | KIAA1505 | KIAA1505 protein | NM_020879 |
| DEPDC6 | DEPDC6,DEP,6,FLJ12428,F LJ13854,DKFZp564B1778 | hypothetical protein FLJ12428 | NM_022783 |
| GLS | GLS,GLS1,FLJ10358,KIAA0 838,DKFZp686O15119 | glutaminase | NM_014905 |
| PPP2R2C | PPP2R2C,PR52,IMYPNO,IM YPNO1,MGC33570 | Homo sapiens protein phosphatase 2 (formerly 2A), regulatory subunit B (PR 52), gamma isoform (PPP2R2C), transcript variant 1, mRNA [M_020416] | NM_020416 |
| NRP1 | NRP1,NRP,CD304,VEGF16 5R,DKFZp781F1414,DKFZp 686A03134 | neuropilin 1 | NM_003873 |
| SP5 | SP5 | Homo sapiens Sp5 transcription factor (SP5), mRNA [M_001003845] | NM_001003 845 |
| ARHGDIB | ARHGDIB,D4,GDIA2,GDID4, LYGDI,Ly-GDI,RAP1GN1 | Rho GDP dissociation inhibitor (GDI) beta | NM_001175 |
| RAI2 | RAI2 | retinoic acid induced 2 | NM_021785 |
| LOC284262 | LOC284262 | hypothetical protein LOC284262 | AL832945 |
| TXNRD3 | TXNRD3,TGR,TR2,TRXR3 | thioredoxin reductase 2 | AF171055 |
| HIVEP1 | HIVEP1,CIRIP,MBP-1,ZNF40,CRYBP1,PRDII-BF1 | human immunodeficiency virus type I enhancer-binding protein 1 | NM_002114 |
| BC042017 | BC042017 | Homo sapiens, clone IMAGE:5311842, mRNA, [BC042017] | BC042017 |
| ABCB2 | TAP1,APT1,PSF1,ABC17,A BCB2,RING4,TAP1N,D6S11 4E,FLJ26666,TAP1*0102N | transporter 1, ATP-binding cassette, sub-family B (MDR/TAP) | NM_000593 |
| GLS | GLS,GLS1,FLJ10358,KIAA0 838,DKFZp686O15119 | Homo sapiens mRNA; cDNA DKFZp686O15119 (from clone DKFZp686O15119), [CR749593] | CR749593 |
| RASSF8 | RASSF8,HoJ-1,C12orf2 | Homo sapiens C120RF2 mRNA for C12ORF2, complete cds, [AB093206] | AB093206 |
| CR622072 | CR622072 | full-length cDNA clone CSODF032YA11 of Fetal brain of Homo sapiens (human), [CR622072] | CR622072 |
| LITAF | LITAF,PIG7,CMT1C,SIMPLE ,TP53I7,FLJ38636,MGC1166 98,MGC116700,MGC116701 ,MGC125274,MGC125275,M GC125276 | LPS-induced TNF-alpha factor | NM_004862 |
| IGF2 | IGF2,INSIGF,pp9974,C11orf 43,FLJ22066,FLJ44734 | Homo sapiens putative insulin-like growth factor II associated protein (LOC492304), mRNA [NM_001007139] | NM_001007 139 |
| CYR61 | CYR61,CCN1,GIG1,IGFBP1 0 | cysteine-rich, angiogenic inducer, 61 | NM_001554 |
| PHF15 | PHF15,JADE2,KIAA0239 | KIAA0239 protein | M_015288 |
| ProSAPiP1 | ProSAPiP1,KIAA0552 | ProSAPiP1 protein | NM_014731 |
| THC2227602 | THC2227602 | 002979 (002979) ORF2280 gene homolog (Fragment), partial (18%) [THC2227602] | |
| LOC389722 | LOC389722,RP11-290L7,1,RP11-138L21,1 | similar to cell recognition molecule CASPR3 | AK054645 |
| ANKRD18A | ANKRD18A,KIAA2015 | Homo sapiens mRNA for KIAA2015 protein, [AB095935] | AB095935 |
| FBN1 | FBN1,FBN,SGS,WMS,MASS ,MFS1,OCTD | fibrillin 1 (Marfan syndrome) | NM_000138 |
| RPS6KA2 | RPS6KA2,RSK,HU-2,RSK3,p90-RSK3,pp90RSK3,MAPKAPK 1C,S6K-alpha,S6K-alpha2 | ribosomal protein S6 kinase, 90kD, polypeptide 2 | NM_021135 |
| GRB10 | GRB10,RSS,IRBP,MEG1,G RB-IR,KIAA0207 | Homo sapiens growth factor receptor-bound protein 10 (GRB10), transcript variant 4, mRNA [NM_001001555] | NM_001001 555 |
| AY336981 | AY336981 | Homo sapiens transcription factor GTF21RD2 isoform 3 (GTF2IRD2) mRNA, complete cds, alternatively spliced, [AY336981] | AY336981 |
| RASSF8 | RASSF8,HoJ-1,C12orf2 | Homo sapiens C12ORF2 mRNA for C12ORF2, complete cds, [AB093206] | AB093206 |
| SLPI | SLPI,ALP,MPI,ALK1,BLPI,H USI,WAP4,WFDC4,HUSI-I | secretory leukocyte protease inhibitor (antileukoproteinase) | NM_003064 |
| SLPI | SLPI,ALP,MPI,ALK1,BLPI,H USI,WAP4,WFDC4,HUSI-I | secretory leukocyte protease inhibitor (antileukoproteinase) | NM_003064 |
| THC2095463 | THC2095463 | Q8LQA6 (Q8LQA6) OJ1125_C04,6 protein, partial (21 %) [THC2095463] | |
| COL16A1 | COL16A1,447AA,FP1572 | collagen, type XVI, alpha 1 | NM_001856 |
| GRAMD3 | GRAMD3,NS3TP2,FLJ21313 | hypothetical protein FLJ21313 | NM_023927 |
| FXYD2 | FXYD2,HOMG2,ATP1G1,M GC12372 | FXYD domain-containing ion transport regulator 2 | NM_021603 |
| PDGFB | PDGFB,SIS,SSV,PDGF2,c-sis,FLJ12858 | platelet-derived growth factor beta polypeptide (simian sarcoma viral (v-sis) oncogene homolog) | NM_002608 |
| FAM107B | FAM107B,C10orf45,FLJ4550 5,MGC11034,MGC90261 | hypothetical protein MGC11034 | NM_031453 |
| LOC440934 | LOC440934 | hypothetical gene supported by BC008048 | BC008048 |
| VCX | VCX,VCX1,VCXB1,VCX-B1,VCX10R,VCX-10r,MGC118975 | variable charge, X chromosome | NM_013452 |
| LAMB3 | LAMB3,LAMNB1 | laminin, beta 3 (nicein (125kD), kalinin (140kD), BM600 (125kD)) | NM_000228 |
| CYR61 | CYR61,CCN1,GIG1,IGFBP1 0 | cysteine-rich, angiogenic inducer, 61 | NM_001554 |
| NCALD | NCALD,MGC33870,MGC748 58 | neurocalcin delta | NM_032041 |
| WNT5A | WNT5A,hWNT5A | wingless-type MMTV integration site family, member 5A | NM_003392 |
| ABCC3 | ABCC3,MLP2,MRP3,ABC31, MOAT-D,cMOAT2,EST90757 | ATP-binding cassette, sub-family C (CFTR/MRP), member 3 | NM_003786 |
| GLIS1 | GLIS1,FLJ36155 | Homo sapiens GLIS family zinc finger 1 (GLIS1), mRNA [NM_147193] | NM_147193 |
| JAG1 | JAG1,AGS,AHD,AWS,HJ1,C D339,JAGL1,MGC104644 | jagged 1 (Alagille syndrome) | NM_000214 |
| NRL | NRL,RP27,D14S46E | neural retina leucine zipper | NM_006177 |
| AGT | AGT,ANHU,SERPINA8 | angiotensinogen (serine (or cysteine) proteinase inhibitor, clade A (alpha-1 antiproteinase, antitrypsin), member 8) | NM_000029 |
| TMSB4X | TMSB4X,FX,TB4X,PTMB4,T MSB4 | Homo sapiens cDNA FLJ31414 fis, clone NT2NE2000260, weakly similar to THYMOSIN BETA-4, | AK055976 |
| CCPG1 | CCPG1,CPR8,KIAA1254 | Homo sapiens cell cycle progression 1 (CCPG1), mRNA [NM_020739] | NM_020739 |
| ADRA2C | ADRA2C,ADRA2L2,ADRAR L2,ADRA2RL2,ALPHA2CAR | adrenergic, alpha-2C-, receptor | NM_000683 |
| BM665539 | BM665539 | UI-E-CL1-afb-b-14-0-UI,s1 UI-E-CL1 Homo sapiens cDNA clone UI-E-CL1-afb-b-14-0-UI 3', mRNA sequence [BM665539] | BM665539 |
| TEX15 | TEX15,DKFZP434M2415 | testis expressed sequence 15 | NM_031271 |
| SEMA3B | SEMA3B,SemA,SEMA5,SE MAA,semaV,LUCA-1,FLJ34863 | sema domain, immunoglobulin domain (lg), short basic domain, secreted, (semaphorin) 3B | NM_004636 |
| NYD-SP18 | NYD-SP18 | testes development-related NYD-SP18 | NM_032599 |
| ASNS | ASNS,TS11 | asparagine synthetase | NM_001673 |
| NFKBIZ | NFKBIZ,IKBZ,INAP,MAIL,FL J30225,FLJ34463 | molecule possessing ankyrin repeats induced by lipopolysaccharide (MAIL), homolog of mouse | NM_031419 |
| AK096677 | AK096677 | Homo sapiens cDNA FLJ39358 fis, clone PEBLM2004015, [AK096677] | AK096677 |
| CA313037 | CA313037 | CA313037 UI-CF-FNO-aex-g-14-0-UI,s1 UI-CF-FNO Homo sapiens cDNA clone UI-CF-FNO-aex-g-14-0-UI 3', mRNA sequence [CA313037] | CA313037 |
| PTPRM | PTPRM,RPTPM,RPTPU,PT PRL1,hR-PTPu,R-PTP-MU | protein tyrosine phosphatase, receptor type, M | NM_002845 |
| SLC1A1 | SLC1A1,EAAC1,EAAT3 | solute carrier family 1 (neuronal/epithelial high affinity glutamate transporter, system Xag), member 1 | NM_004170 |
| GRB10 0 | GRB10,RSS,IRBP,MEG1,G RB-IR,KIAA0207 | Homo sapiens growth factor receptor-bound protein 10 (GRB10), transcript variant 4, mRNA [NM_001001555] | NM_001001 555 |
| NQO1 | NQO1,DTD,QR1,DHQU,DIA 4,NMOR1,NMOR1 | diaphorase (NADH/NADPH) (cytochrome b-5 reductase) | NM_000903 |
| A_24_P40105 1 | A_24_P401051 | Unknown | |
| GPR161 | GPR161,RE2,FLJ33952 | Homo sapiens cDNA FLJ33952 fis, clone CTONG2018614, [AK091271] | AK091271 |
| A_32_P32905 | A_32_P32905 | Unknown | |
| LOC389652 | LOC389652 | similar to asparagine synthetase; glutamine-dependent asparagine synthetase; TS11 cell cycle control protein | XM_372040 |
| SRGAP3 | SRGAP3,WRP,MEGAP,SRG AP2,ARHGAP14,KIAA0411 | Homo sapiens SLIT-ROBO Rho GTPase activating protein 3 (SRGAP3), mRNA [NM_014850] | NM_014850 |
| PDE4D | PDE4D,DPDE3,STRK1,HSP DE4D,PDE4DN2 | phosphodiesterase 4D, cAMP-specific (dunce (Drosophila)-homolog phosphodiesterase E3) | NM_006203 |
| THC2055165 | THC2055165 | Unknown | |
| LOC63920 | LOC63920 | transposon-derived Buster3 transposase-like | NM_022090 |
| AK022020 | AK022020 | Homo sapiens cDNA FLJ11958 fis, clone HEMBB1000996, [AK022020] | AK022020 |
| MGAT4A | MGAT4A,GNT-IV,GNT-IVA | mannosyl (alpha-1,3-)-glycoprotein beta-1,4-N-acetylglucosaminyltransferase, isoenzyme A | NM_012214 |
| THC2201936 | THC2201936 | Q8WSI5 (Q8WSI5) Prophenol oxidase, partial (3%) [THC2201936] | |
| THC2091303 | THC2091303 | GRI1_HUMAN (Q9Y3R0) Glutamate receptor-interacting protein 1 (GRIP1 protein), partial (82%) [THC2091303] | |
| FCRL2 | FCRL2 | Homo sapiens hypothetical protein FLJ31052 (FLJ31052), mRNA [NM_152378] | NM_152378 |
| PDE4DIP | PDE4DIP,MMGL,CMYA2,M GC75440,DKFZp781J054 | Homo sapiens phosphodiesterase 4D interacting protein (myomegalin) (PDE4DIP), transcript variant 5, mRNA [NM_001002811] | NM_001002 811 |
| WBP5 | WBP5,DKFZp313K1940 | pp21 homolog | NM_016303 |
| PIM1 | PIM1,PIM | pim-1 oncogene | NM_002648 |
| NUPR1 | NUPR1,P8,COM1 | nuclear protein 1 | NM_012385 |
| SMAD7 | SMAD7,MADH7,MADH8,FLJ 16482 | MAD (mothers against decapentaplegic, Drosophila) homolog 7 | NM_005904 |
| LOC63920 | LOC63920 | transposon-derived Buster3 transposase-like | NM_022090 |
| STAT1 | STAT1,ISGF-3,STAT91,DKFZp686B04100 | signal transducer and activator of transcription 1, 91kD | NM_007315 |
| AK057151 | AK057151 | Homo sapiens cDNA FLJ32589 fis, clone SPLEN2000443, [AK057151] | AK057151 |
| PPM1H | PPM1H,ARHCL1,FLJ13253, KIAA1157 | KIAA1157 protein | AB032983 |
| BM806490 | BM806490 | AGENCOURT_6553853 NIH_MGC_71 Homo sapiens cDNA clone IMAGE:5555887 5', mRNA sequence [BM806490] | BM806490 |
| AL390181 | AL390181 | Homo sapiens mRNA; cDNA DKFZp547J125 (from clone DKFZp547J125), [AL390181] | AL390181 |
| LOC150356 | LOC150356 | hypothetical protein BC012882 | BC012882 |
| WNT7B | WNT7B | Homo sapiens wingless-type MMTV integration site family, member 7B (WNT7B), mRNA [NM_058238] | NM_058238 |
| BF210146 | BF210146 | 601874052F1 NIH_MGC_54 Homo sapiens cDNA clone IMAGE:4098852 5', mRNA sequence [BF210146] | BF210146 |
| LRP11 | LRP11,MANSC3,FLJ14735, MGC39092, bA350J20,3 | hypothetical protein FLJ14735 | NM_032832 |
| FGFR2 | FGFR2,BEK,JWS,CEK3,CF D1,ECT1,KGFR,TK14,TK25, BFR-1,K-SAM | fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome) | NM_022972 |
| MT2A | MT2A,MT2 | metallothionein 2A | NM_005953 |
| BF378046 | BF378046 | BF378046 RC1-TN0151-270900-013-b06 TN0151 Homo sapiens cDNA, mRNA sequence [BF378046] | BF378046 |
| MT2A | MT2A,MT2 | metallothionein 2A | BC007034 |
| BC037328 | BC037328 | Homo sapiens cDNA clone IMAGE:5263455, partial cds, [BC037328] | BC037328 |
| MT2A | MT2A,MT2 | metallothionein 2A | NM_005953 |
| ZNF323 | ZNF323,ZNF310P,FLJ23407 ,ZNF20-Lp,dJ874C20,2 | hypothetical protein FLJ23407 | NM_030899 |
| TBC1D8 | TBC1D8,AD3,VRP,HBLP1 | vascular Rab-GAP/TBC-containing | NM_007063 |
| BLVRA | BLVRA,BLVR,BVRA | biliverdin reductase A | NM_000712 |
| FGFR2 | FGFR2,BEK,JWS,CEK3,CF D1,ECT1,KGFR,TK14,TK25, BFR-1,K-SAM | fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome) | NM_022973 |

**Table 2 : List of the under-expressed genes (at least two-fold) in the docetaxel resistant cell-lines.**

| **Primary Sequence Name** | **Sequence Name(s)** | **Sequence Description** | **Accession #** |
|---|---|---|---|
| PIK3C3 | PIK3C3,Vps34,MGC61518 | phosphoinositide-3-kinase, class 3 | NM_002647 |
| SCARB1 | SCARB1,CLA1,SRB1,CLA-1,SR-BI,CD36L1,MGC138242 | CD36 antigen (collagen type I receptor, thrombospondin receptor)-like 1 | NM_005505 |
| ASGR1 | ASGR1,ASGPR,CLEC4H1,H s,12056 | asialoglycoprotein receptor 1 | M_001671 |
| FLJ22659 | FLJ22659 | hypothetical protein FLJ22659 | AK026312 |
| ASMTL | ASMTL,ASTML,ASMTLX,AS MTLY | acetylserotonin O-methyltransferase-like | NM_004192 |
| ARHGAP10 0 | ARHGAP10,GRAF2,PS-GAP,FLJ20896,FLJ41791 | hypothetical protein FLJ20896 | NM_024605 |
| EDG6 | EDG6,LPC1,S1P4,SLP4,S1P R4 | endothelial differentiation, G-protein-coupled receptor 6 | NM_003775 |
| KCNC3 | KCNC3,KV3,3,SCA13,KSHIII D | potassium voltage-gated channel, Shaw-related subfamily, member 3 | NM_004977 |
| MGC11332 | MGC11332 | hypothetical protein MGC11332 | M_032718 |
| NRG1 | NRG1,GGF,HGL,HRG,NDF, ARIA,GGF2,HRG1,HRGA,S MDF | neuregulin 1 | NM_004495 |
| LOC339240 | LOC339240 | Homo sapiens keratin pseudogene (LOC339240) on chromosome 17 [NRL_001443] | NR_001443 |
| PTPN3 | PTPN3,PTPH1,DKFZp686N0 569 | Homo sapiens mRNA; cDNA DKFZp686N0569 (from clone DKFZp686N0569), [CR749204] | CR749204 |
| AK123483 | AK123483 | Homo sapiens cDNA FLJ41489 fis, clone BRTHA2004582, [AK123483] | AK123483 |
| NRG1 | NRG1,GGF,HGL,HRG,NDF, ARIA,GGF2,HRG1,HRGA,S MDF | neuregulin 1 | NM_013962 |
| FAM80A | FAM80A,MGC47816,RP11-157D18,1 | Homo sapiens hypothetical protein MGC47816 (MGC47816), mRNA [NM_173642] | NM_173642 |
| BAMBI | BAMBI,NMA | putative transmembrane protein | NM_012342 |
| PTPN3 | PTPN3,PTPH1,DKFZp686N0 569 | protein tyrosine phosphatase, non-receptor type 3 | NM_002829 |
| SAMD8 | SAMD8,FLJ25082 | Homo sapiens sterile alpha motif domain containing 8 (SAMD8), mRNA [NM_144660] | NM_144660 |
| KCNMB4 | KCNMB4 | potassium large conductance calcium-activated channel, subfamily M, beta member 4 | NM_014505 |
| SPG20 | SPG20,SPARTIN,TAHCCP1, KIAA0610 | Homo sapiens spastic paraplegia 20, spartin (Troyer syndrome) (SPG20), mRNA [NM_015087] | NM_015087 |
| RGS16 | RGS16,RGS-R,A28-RGS14,A28-RGS14P | regulator of G-protein signalling 16 | NM_002928 |
| UGT2B7 | UGT2B7,UGT2B9 | UDP glycosyltransferase 2 family, polypeptide B7 | NM_001074 |
| LMBRD2 | LMBRD2,MGC125692,DKFZ p434H2226,DKFZp686G105 7 | Homo sapiens hypothetical protein DKFZp434H2226 (DKFZp434H2226), mRNA [NM_001007527] | NM_001007 527 |
| TMPRSS4 | TMPRSS4,MT-SP2,TMPRSS3 | transmembrane protease, serine 4 | NM_019894 |
| PDK1 | PDK1 | pyruvate dehydrogenase kinase, isoenzyme 1 | NM_002610 |
| RAB39B | RAB39B | Homo sapiens RAB39B, member RAS oncogene family (RAB39B), mRNA [NM_171998] | NM_171998 |
| HSPH1 | HSPH1,HSP105A,HSP105B, KIAA0201,NY-CO-25, DKFZp686M05240 | heat shock 105kD | NM_006644 |
| PSCDBP | PSCDBP,HE,B3-1,CASP,CYBR,CYTIP | pleckstrin homology, Sec7 and coiled/coil domains, binding protein | NM_004288 |
| UGT2B11 | UGT2B11,MGC129611,MGC 129612 | UDP glycosyltransferase 2 family, polypeptide B11 | NM_001073 |
| ZNF516 | ZNF516,HsT287 | KIAA0222 gene product | D86975 |
| CKB | CKB,B-CK,CKBB | creatine kinase, brain | NM_001823 |
| SLC7A8 | SLC7A8,LAT2,LPI-PC1 | Homo sapiens solute carrier family 7 (cationic amino acid transporter, y+ system), member 8 (SLC7A8), transcript variant 2, mRNA [NM_182728] | NM_182728 |
| UGT2B28 | UGT2B28 | Homo sapiens UDP glycosyltransferase 2 family, polypeptide B28 (UGT2B28), mRNA, | NM_053039 |
| SEMA3C | SEMA3C,SemE,SEMAE | sema domain, immunoglobulin domain (lg), short basic domain, secreted, (semaphorin) 3C | NM_006379 |
| PDK1 | PDK1 | pyruvate dehydrogenase kinase, isoenzyme 1 | NM_002610 |
| GATA2 | GATA2,NFE1B,MGC2306 | hypothetical protein MGC2306 | NM_032638 |
| THC2064535 | THC2064535 | Unknown | |
| PDLIM5 | PDLIM5,L9,ENH,LIM | LIM protein (similar to rat protein kinase C-binding enigma) | NM_006457 |
| BX538293 | BX538293 | Homo sapiens mRNA; cDNA DKFZp686F09157 (from clone DKFZp686F09157), [BX538293] | BX538293 |
| HYAL1 | HYAL1,NAT6,LUCA1,HYAL-1,MGC45987 | Homo sapiens hyaluronoglucosaminidase 1 (HYAL1), transcript variant 4, mRNA [NM_153284] | NM_153284 |
| UGT2B4 | UGT2B4,UGT2B11 | UDP glycosyltransferase 2 family, polypeptide B4 | NM_021139 |
| THC2185385 | THC2185385 | Unknown | |
| FLJ10159 | FLJ10159 | hypothetical protein FLJ10159 | AK001021 |
| UGT2B10 | UGT2B10,MGC142209 | UDP glycosyltransferase 2 family, polypeptide B10 | NM_001075 |
| PRTFDC1 | PRTFDC1,HHGP,FLJ11888 | HHGP protein | NM_020200 |
| A_24_P57526 7 | A_24_P575267 | Unknown | |
| PTGES | PTGES,PGES,PIG12,PP102, PP1294,MGST-IV,MGST1 L1,TP53112,MGC1 0317,MGST1-L1 | prostaglandin E synthase | NM_004878 |
| DNAJC15 | DNAJC15,MCJ,HSD18,DNAJD1 | DNAJ domain-containing | NM_013238 |
| NPAS1 | NPAS1,MOP5,PASD5 | neuronal PAS domain protein 1 | NM_002517 |
| A_24_P47894 0 | A_24_P478940 | Unknown | |
| FRMD4B | FRMD4B,GRSP1,KIAA1013, 6030440G05Rik | Homo sapiens FERM domain containing 4B, mRNA (cDNA clone IMAGE:4508579), partial cds, [BC028291] | BC028291 |
| SLC7A11 | SLC7A11,xCT,CCBR1 | Homo sapiens solute carrier family 7, (cationic amino acid transporter, y+ system) member 11, mRNA (cDNA clone IMAGE:5300264), partial cds, [BC041925] | BC041925 |
| AF132203 | AF132203 | Homo sapiens PRO1933 mRNA, complete cds, [AF132203] | AF132203 |
| FLJ43855 | FLJ43855 | similar to sodium- and chloride-dependent creatine transporter | NM_198857 |
| TFDP2 | TFDP2,DP2,Dp-2 | transcription factor Dp-2 (E2F dimerization partner 2) | NM_006286 |
| PFKFB4 | PFKFB4 | 6-phosphofructo-2-kinase/fructose-2,6-biphosphatase 4 | NM_004567 |
| TAC3 | TAC3,NKB,NKNB,PRO1155, ZNEUROK1 | tachykinin 3 (neuromedin K, neurokinin beta) | NM_013251 |
| ENC1 | ENC1,NRPB,CCL28,ENC-1,PIG10,TP53I10,FLJ39259 | ectodermal-neural cortex (with BTB-like domain) | NM_003633 |
| EPAS1 | EPAS1,HLF,MOP2,HIF2A,PA SD2 | endothelial PAS domain protein 1 | NM_001430 |
| NRP2 | NRP2,NP2,NPN2,PRO2714, MGC126574,VEGF165R2 | Homo sapiens neuropilin 2 (NRP2), transcript variant 6, mRNA [M_201264] | NM_201264 |
| MFHAS1 | MFHAS1,MASL1,FLJ23354 | MFH-amplified sequences with leucine-rich tandem repeats 1 | NM_004225 |
| AK024680 | AK024680 | Homo sapiens cDNA: FLJ21027 fis, clone CAE07110, [AK024680] | AK024680 |
| SYTL3 | SYTL3,SLP3,MGC105130,M GC118883,MGC118884,MG C118885 | Homo sapiens synaptotagmin-like 3 (SYTL3), mRNA [NM_001009991] | NM_001009 991 |
| BDNF | BDNF,MGC34632 | Homo sapiens brain-derived neurotrophic factor (BDNF), transcript variant 6, mRNA [NM_170734] | NM_170734 |
| AL137342 | AL137342 | Homo sapiens mRNA; cDNA DKFZp761G1111 (from clone DKFZp761G 1111 [AL137342] | AL137342 |
| D4S234E | D4S234E,P21,NSG1,D4S234 ,NEEP21 | DNA segment on chromosome 4 (unique) 234 expressed sequence | NM_014392 |
| LCP1 | LCP1,CP64,PLS2,LC64P,FL J25423,FLJ26114,FLJ39956, L-PLASTIN,DKFZp781A23186 | lymphocyte cytosolic protein 1 (L-plastin) | NM_002298 |
| A_32_P95067 | A_32_P95067 | Unknown | |
| THC2038567 | THC2038567 | Q7UFH6 (Q7UFH6) Acetyl-CoA carboxylase (Biotin carboxyl carrier subunit) accB, partial (16%) [THC2038567] | |
| LMO1 | LMO1,TTG1,RBTN1,RHOM1, MGC116692 | LIM domain only 1 (rhombotin 1) | NM_002315 |
| BSPRY | BSPRY,FLJ20150 | hypothetical protein FLJ20150 | M_017688 |
| BDNF | BDNF,MGC34632 | Homo sapiens brain-derived neurotrophic factor (BDNF), transcript variant 1, mRNA [NM_170735] | NM_170735 |
| UGT8 | UGT8,CGT | UDP glycosyltransferase 8 (UDP-galactose ceramide galactosyltransferase) | NM_003360 |
| LIPG | LIPG,EL,EDL,PR0719 | lipase, endothelial | NM_006033 |
| AW205591 | AW205591 | UI-H-BI1-afr-b-02-0-UI,s1 NCI_CGAP_Sub3 Homo sapiens cDNA clone IMAGE:2722515 3', mRNA sequence [AW205591] | AW205591 |
| AKAP12 | AKAP12,AKAP250,DKFZp68 6M0430, DKFZp686O0331 | A kinase (PRKA) anchor protein (gravin) 12 | NM_005100 |
| B3GALT1 | B3GALT1,MGC126594,beta3 Gal-T1 | UDP-Gal:betaGlcNAc beta 1,3-galactosyltransferase, polypeptide 1 | NM_020981 |
| CHST7 | CHST7,C6ST-2 | carbohydrate (N-acetylglucosamine 6-O) sulfotransferase 7 | NM_019886 |
| NMNAT2 | NMNAT2,PNAT2,PNAT-2,C1orf15,MGC2756,KIAA04 79 | Homo sapiens nicotinamide nucleotide adenylyltransferase 2 (NMNAT2), transcript variant 1, mRNA [NM_015039] | NM_015039 |
| SLC12A3 | SLC12A3,TSC,NCCT | solute carrier family 12 (sodium/chloride transporters), member 3 | NM_000339 |
| SLC22A2 | SLC22A2,OCT2,MGC32628 | solute carrier family 22 (organic cation transporter), member 2 | NM_003058 |
| LMBRD2 | LMBRD2,MGC125692,DKFZ p434H2226,DKFZp686G105 7 | Homo sapiens hypothetical protein DKFZp434H2226 (DKFZp434H2226), mRNA [NM_001007527] | NM_001007 527 |
| SCD | SCD,SCD1,FADS5,PRO0998 | stearoyl-CoA desaturase (delta-9-desaturase) | NM_005063 |
| AMPH | AMPH,AMPH1 | amphiphysin (Stiff-Mann syndrome with breast cancer 128kD autoantigen) | NM_001635 |
| ANKRD37 | ANKRD37,Lrp2bp,MGC1115 07 | Homo sapiens low density lipoprotein receptor-related protein binding protein (Lrp2bp), mRNA[NM_181726] | NM_181726 |
| LIN7A | LIN7A,LIN7,VELI1,LIN-7A,MALS-1,TIP-33,MGC148143 | Vertebrate LIN7 homolog 1, Tax interaction protein 33 | NM_004664 |
| PHEX | PHEX,HYP,PEX,XLH,HPDR, HYP1,HPDR1 | phosphate regulating gene with homologies to endopeptidases on the X chromosome (hypophosphatemia, vitamin D resistant rickets) | NM_000444 |
| C1QL1 | C1QL1,CRF,C1QRF | C1q-related factor | NM_006688 |
| EPAS1 | EPAS1,HLF,MOP2,HIF2A,PA SD2 | endothelial PAS domain protein 1 | NM_001430 |
| KCNC4 | KCNC4,KV3,4,KSHIIIC,HKS HIIIC,MGC126818 | Homo sapiens potassium voltage-gated channel, Shaw-related subfamily, member 4 (KCNC4), transcript variant 2, mRNA [NM_153763] | NM_153763 |
| FOXL2 | FOXL2,BPES,PFRK,POF3,B PES1,PINTO | forkhead box L2 | NM_023067 |
| SCD | SCD,SCD1,FADS5,PRO0998 | stearoyl-CoA desaturase (delta-9-desaturase) | NM_005063 |
| FGFBP1 | FGFBP1,FGFBP,HBP17 7 | heparin-binding growth factor binding protein | M_005130 |
| CASP8 | CASP8,CAP4,MACH,MCH5, FLICE,MGC78473 | caspase 8, apoptosis-related cysteine protease | NM_033356 |
| LZTS1 | LZTS1,F37,FEZ1 | leucine zipper, putative tumor suppressor1 | NM_021020 |
| SYTL3 | SYTL3,SLP3,MGC105130,M GC118883,MGC118884,MG C118885 | Homo sapiens synaptotagmin-like 3 (SYTL3), mRNA [NM_001009991] | NM_001009 991 |
| HSHPX5 | HSHPX5 | PREDICTED: Homo sapiens HPX-5 (HSHPX5), mRNA [XM_496232] | XM_496232 |
| NLGN1 | NLGN1,KIAA1070,MGC4511 5 | neuroligin 1 | NM_014932 |
| MGST1 | MGST1,MGST,GST12,MGST -I,MGC14525 | Homo sapiens microsomal glutathione S-transferase 1 (MGST1), transcript variant 1c, mRNA [NM_145791] | NM_145791 |
| PLXNA2 | PLXNA2,OCT,PLXN2,FLJ11 751,FLJ30634,KIAA0463 | plexin A2 | NM_025179 |
| ST8SIA4 | ST8SIA4,PST,PST1,SIAT8D, MGC34450,MGC61459,ST8 SIA-IV | sialyltransferase 8 (alpha-2, 8-polysialytransferase) D | NM_005668 |
| THC2050576 | THC2050576 | Unknown | |
| SLC3A1 | SLC3A1,D2H,ATR1,NBAT,R BAT,CSNU1,FLJ34681 | solute carrier family 3 (cystine, dibasic and neutral amino acid transporters, activator of cystine, dibasic and neutral amino acid transport), member 1 | NM_000341 |
| TNRC9 | TNRC9,CAGF9 | trinucleotide repeat containing 9 | U80736 |
| AK022997 | AK022997 | Homo sapiens cDNA FLJ12935 fis, clone NT2RP2004982, [AK022997] | AK022997 |
| UGT8 | UGT8,CGT | UDP glycosyltransferase 8 (UDP-galactose ceramide galactosyltransferase) | U62899 |
| LETM2 | LETM2,FLJ25409 | Homo sapiens leucine zipper-EF-hand containing transmembrane protein 2 (LETM2), mRNA [NM_144652] | NM_144652 |
| PLAC8 | PLAC8,C15,onzin | hypothetical protein | NM_016619 |
| DIAPH2 | DIAPH2,DIA,POF,DIA2,POF 2,FLJ11167 | diaphanous (Drosophila, homolog) 2 | NM_007309 |
| BC014452 | BC014452 | Homo sapiens cDNA clone IMAGE:4903661, complete cds, [BC014452] | BC014452 |
| SUNC1 | SUNC1,MGC33329 | Homo sapiens Sad1 and UNC84 domain containing 1 (SUNC1),mRNA [NM_152782] | NM_152782 |
| DUSP13 | DUSP13,BEDP,MDSP,TMDP ,SKRP4,FLJ32450 | protein phosphatase | NM_016364 |
| AUTS2 | AUTS2,KIAA0442,MGC1314 0 | Homo sapiens autism susceptibility candidate 2 (AUTS2), mRNA [M_015570] | NM_015570 |
| PLAC8 | PLAC8,C15,onzin | hypothetical protein | NM_016619 |
| THC2210862 | THC2210862 | Unknown | |
| MSX2 | MSX2,FPP,MSH,PFM,CRS2, HOX8,PFM1 | msh (Drosophila) homeo box homolog 2 | NM_002449 |
| SMAD9 | SMAD9,MADH6,MADH9,SM AD8A,SMAD8B | MAD (mothers against decapentaplegic, Drosophila) homolog 9 | NM_005905 |
| TTN | TTN,TMD,CMH9,CMD1G,CM PD4,HMERF,LGMD2J,FLJ26 020,FLJ26409,FLJ32040,FLJ 34413,FLJ39564,FLJ43066,D KFZp451N061 | Homo sapiens titin (TTN), transcript variant N2-A, mRNA [NM_133378] | NM_133378 |
| LRRN6C | LRRN6C,LERN3,LINGO2,FL J31810 0 | Homo sapiens hypothetical protein FLJ31810 (FLJ31810), mRNA [NM_152570] | NM_152570 |
| MEIS2 | MEIS2,MRG1,MGC2820,HsT 18361 | Homo sapiens Meis1, myeloid ecotropic viral integration site 1 homolog 2 (mouse) (MEIS2), transcript variant a, mRNA [NM_170677] | NM_170677 |
| DHRS3 | DHRS3,SDR1,RDH17,Rsdr1, retSDR1 | short-chain dehydrogenase/reductase 1 | M_004753 |
| OLR1 | OLR1,LOX1,CLEC8A,SCAR E1 | oxidised low density lipoprotein (lectin-like) receptor 1 | NM_002543 |
| NSBP1 | NSBP1 | nucleosomal binding protein 1 | NM_030763 |
| MOXD1 | MOXD1,MOX,PRO5780,dJ24 8E1,1,DKFZP564G202 | Homo sapiens monooxygenase, DBH-like 1 (MOXD1), mRNA [NM_015529] | NM_015529 |
| DCAMKL1 | DCAMKL1,DCLK,KIAA0369 | doublecortin and CaM kinase-like 1 | NM_004734 |
| C12orf59 | C12orf59,FLJ31166,MGC111 385 | hypothetical protein FLJ31166 | NM_153022 |
| A_23_P13685 7 | A_23_P136857 | Unknown | |
| BF675806 | BF675806 | 602083723F1 NIH_MGC_83 Homo sapiens cDNA clone IMAGE:4248004 5', mRNA sequence [BF675806] | BF675806 |
| SLC44A5 | SLC44A5,CTL5,FLJ34081,M GC34032 | Homo sapiens hypothetical protein MGC34032 (MGC34032), mRNA [NM_152697] | NM_152697 |
| SALL1 | SALL1,TBS,HSAL1,ZNF794 | sal (Drosophila)-like 1 | NM_002968 |
| GPR177 | GPR177,MRP,WLS,C1orf139 ,FLJ23091,MGC14878,MGC 131760 | hypothetical protein FLJ23091 | NM_024911 |
| AK3L1 | AK3L1,AK3,AK4 | Homo sapiens cDNA: FLJ23313 fis, clone HEP11919 | AK026966 |
| AK3L1 | AK3L1,AK3,AK4 | adenylate kinase 3 | NM_013410 |
| FZD8 | FZD8,FZ-8,hFZ8 | frizzled (Drosophila) homolog 8 | NM_031866 |
| THC2088463 | THC2088463 | ALU7_HUMAN (P39194) Alu subfamily SQ sequence contamination warning entry, partial (8%) [THC2088463] | |
| FLJ39502 | FLJ39502,FLJ26337,MGC13 4803 | hypothetical protein FLJ39502 | AK096821 |
| PROS1 | PROS1,PSA,PROS,PS21,PS 22,PS23,PS24,PS25,PS 26,Protein S,protein Sa | protein S (alpha) | NM_000313 |
| PTPRD | PTPRD,HPTP,PTPD,HPTPD, MGC119750,MGC119751,M GC119752,MGC119753,HPT P-DELTA,R-PTP-DELTA | protein tyrosine phosphatase, receptor type, D | NM_002839 |
| PDE1A | PDE1A,HCAM1,HSPDE1A,M GC26303 | phosphodiesterase 1A, calmodulin-dependent | NM_005019 |
| MYB | MYB,efg,c-myb,c-myb_CDS | v-myb avian myeloblastosis viral oncogene homolog | NM_005375 |
| SLC16A10 | SLC16A10,TAT1,PR00813 | hypothetical protein PRO0813 | NM_018593 |
| GJA7 | GJA7,CX45,DKFZp686P073 8 | gap junction protein, alpha 7, 45kD (connexin 45) | NM_005497 |
| GAL | GAL,GALN,GLNN,MGC4016 7 | galanin-related peptide | NM_015973 |
| CPM | CPM | carboxypeptidase M | NM_001874 |
| PDE1A | PDE1A,HCAM1,HSPDE1A,M GC26303 | phosphodiesterase 1A, calmodulin-dependent | NM_005019 |
| PLXNA2 | PLXNA2,OCT,PLXN2,FLJ11 751,FLJ30634,KIAA0463 | Homo sapiens cDNA FLJ30634 fis, clone CTONG2002453, | AK055196 |
| PDE1A | PDE1A,HCAM1,HSPDE1A,M GC26303 | Homo sapiens phosphodiesterase 1A, calmodulin-dependent (PDE1A), transcript variant 2, mRNA [NM_001003683] | NM_001003 683 |
| AW467174 | AW467174 | AW467174 ha35g06,x1 NCI_CGAP_Kid12 Homo sapiens cDNA clone IMAGE:2875738 3' similar to gb:X60673_rna1 GTP:AMP PHOSPHOTRANSFERASE MITOCHONDRIAL (HUMAN);, mRNA sequence [AW467174] | AW467174 |
| PLAT | PLAT,TPA,T-PA,DKFZp686103148 | plasminogen activator, tissue | NM_000930 |
| LOC441047 | LOC441047 | similar to Adenylate kinase isoenzyme 4, mitochondrial (ATP-AMP transphosphorylase) | XM_496720 |
| CXCR4 | CXCR4,FB22,HM89,LAP3,L CR1,NPYR,WHIM,CD184,LE STR,NPY3R,NPYRL,HSY3R R,NPYY3R,D2S201 E | chemokine (C-X-C motif), receptor 4 (fusin) | NM_003467 |
| AK3L1 | AK3L1,AK3,AK4 | adenylate kinase 3 | NM_013410 |
| SMPDL3A | SMPDL3A,ASM3A,ASML3a, FLJ20177,yR36GH4,1 | Homo sapiens sphingomyelin phosphodiesterase, acid-like 3A (SMPDL3A), mRNA [M_006714] | M_006714 |
| KIAA0960 | KIAA0960 | KIAA0960 protein | AB023177 |
| LHFP | LHFP,MGC22429 | lipoma HMGIC fusion partner | NM_005780 |
| CPM | CPM | carboxypeptidase M | NM_001874 |
| A_24_P34529 | A_24_P345290 | Unknown | |
| RXFP1 | RXFP1,LGR7,LGR7,1,LGR7, 2,LGR7,10,MGC138347,MG C142177 | leucine-rich repeat-containing G protein-coupled receptor 7 | M_021634 |
| PNOC | PNOC,PPNOC | prepronociceptin | NM_006228 |
| GALNT14 | GALNT14,GALNT15,FLJ126 91,FLJ13977,GaINac-T10,GaINac-T14 | hypothetical protein FLJ12691 | NM_024572 |
| TM4SF1 | TM4SF1,L6,H-L6,M3S1,TAAL6 | Homo sapiens transmembrane 4 superfamily member 1 (TM4SF1), mRNA[NM_014220] | M_014220 |
| ZAR1 | ZAR1 | Homo sapiens zygote arrest 1 (ZAR1), mRNA[NM_175619] | NM_175619 |
| A_23_P10091 | A_23_P10091 | Unknown | |
| GLT8D2 | GLT8D2,FLJ31494 | gycosyltransferase | M_031302 |
| RXFP1 | RXFP1,LGR7,LGR7,1,LGR7, 2,LGR7,10,MGC138347,MG C142177 | Relaxin receptor 1 (Leucine-rich repeat-containing G protein-coupled receptor 7), [Source:Uniprot/SWISSPROT;Acc:Q9H BX9] [ENST00000343542] | BX647985 |
| CGNL1 | CGNL1,JACOP,FLJ14957,KI AA1749,MGC138254 | hypothetical protein FLJ14957 | NM_032866 |
| AK094972 | AK094972 | Homo sapiens cDNA FLJ37653 fis, clone BRHIP2010217, [AK094972] | AK094972 |
| LRCH2 | LRCH2,KIAA1495,dA204F4,4 | Homo sapiens leucine-rich repeats and calponin homology (CH) domain containing 2 (LRCH2), mRNA [NM_020871] | NM_020871 |
| BM930757 | BM930757 | UI-E-EJ1-ajm-I-20-0-UI,r1 UI-E-EJ1 Homo sapiens cDNA clone UI-E-EJ1-ajm-I-20-0-UI 5', mRNA sequence [BM930757] | BM930757 |
| ATP8A1 | ATP8A1,ATPIA,ATPP2,ATPA SEII,MGC26327,MGC130042 ,MGC130043 | Homo sapiens ATPase, aminophospholipid transporter (APLT), Class I, type 8A, member 1 (ATP8A1), mRNA [NM_006095] | NM_006095 |
| SOX9 | SOX9,CMD1,SRA1,CMPD1 | SRY (sex determining region Y)-box 9 (campomelic dysplasia, autosomal sex-reversal) | NM_000346 |
| SLC39A8 | SLC39A8,BIGM103,LZT-Hs6 | up-regulated by BCG-CWS | NM_022154 |
| TMEM47 | TMEM47,BCMP1,TM4SF10, MGC32949,DKFZp564E153, DKFZP761J17121 | brain cell membrane protein 1 | M_031442 |
| SLC10A4 | SLC10A4,P4,MGC29802 | Homo sapiens solute carrier family 10 (sodium/bile acid cotransporter family), member 4 (SLC10A4), mRNA [NM_152679] | NM_152679 |
| SLC1A3 | SLC1A3,EA6,EAAT1,GLAST, GLAST1,FLJ25094 | solute carrier family 1 (glial high affinity glutamate transporter), member 3 | M_004172 |
| EDG7 | EDG7 | Homo sapiens cDNA FLJ34412 fis, clone HEART2002432, [AK091731] | AK091731 |
| ITGA2 | ITGA2,BR,GPla,CD49B,VLA-2,VLAA2 | integrin, alpha 2 (CD49B, alpha 2 subunit of VLA-2 receptor) | NM_002203 |
| SLC1A3 | SLC1A3,EA6,EAAT1,GLAST, GLAST1,FLJ25094 | solute carrier family 1 (glial high affinity glutamate transporter), member 3 | M_004172 |
| PLCXD3 | PLCXD3 | Homo sapiens phosphatidylinositol-specific phospholipase C, X domain containing 3 (PLCXD3), mRNA [M_001005473] | NM_001005 473 |
| BF514799 | BF514799 | UI-H-BW1-anj-a-01-0-UI,s1 NCI_CGAP_Sub7 Homo sapiens cDNA clone IMAGE:3082272 3', mRNA sequence [BF514799] | BF514799 |
| SLC16A12 | SLC16A12,MCT12 | Homo sapiens cDNA FLJ42911 fis, clone BRHIP3024118, weakly similar to Monocarboxylate transporter 4, [AK124901] | AK124901 |
| THC2208430 | THC2208430 | Unknown | |
| THC2182743 | THC2182743 | EDG7_HUMAN (Q9UBY5) Lysophosphatidic acid receptor Edg-7 (LPA receptor 3) (LPA-3), partial (36%) [THC2182743] | |
| C4orf18 | C4orf18,AD021,AD036,FLJ3 8155,DKFZp434L142 | AD021 protein | NM_016613 |
| ANKRD38 | ANKRD38,FLJ10884,KIAA01 72,dJ1078M7,1,RP5-1155K23,5 | Homo sapiens hypothetical protein LOC163782 (LOC163782), mRNA [NM_181712] | NM_181712 |
| CALCRL | CALCRL,CRLR,CGRPR | calcitonin receptor-like | NM_005795 |
| hSHISA3 | LOC152573 | hypothetical protein BC012029 | BC012029 |

**Table 3 : List of the over-expressed genes by at least two fold in the docetaxel resistant cell-lines.**

| | **Log(Ratio) for each dose** | | | | **Log** **(ratio)** | **Ratio** | **Fold change** |
|---|---|---|---|---|---|---|---|
| Primary Sequence Name | **50nM** | **25nM** | **12nM** | **5nM** | **Mean** | **Mean** | **Mean** |
| RPIB9 | 1,49 | 1,51 | 1,55 | 1,59 | 1,53 | 34,22 | 34,22 |
| CXCL2 | 1,45 | 1,28 | 1,96 | 1,28 | 1,49 | 30,98 | 30,98 |
| AL137761 | 1,18 | 1,13 | 1,53 | 1,54 | 1,35 | 22,17 | 22,17 |
| TFPI2 | 1,61 | 1,65 | 1,48 | 0,61 | 1,34 | 21,84 | 21,84 |
| THC2051204 | 1,32 | 1,21 | 1,34 | 1,34 | 1,30 | 20,11 | 20,11 |
| TNF | 1,22 | 1,28 | 1,52 | 1,04 | 1,26 | 18,36 | 18,36 |
| ABCB1 | 1,21 | 1,19 | 1,20 | 1,20 | 1,20 | 15,83 | 15,83 |
| PURG | 1,34 | 1,23 | 1,11 | 1,07 | 1,19 | 15,39 | 15,39 |
| ADAMTS5 | 1,22 | 1,34 | 1,03 | 0,96 | 1,14 | 13,75 | 13,75 |
| MCTP1 | 1,01 | 1,14 | 1,28 | 1,00 | 1,11 | 12,79 | 12,79 |
| SPTLC2L | 0,88 | 1,22 | 1,36 | 0,92 | 1,10 | 12,46 | 12,46 |
| OAS3 | 1,15 | 1,13 | 1,09 | 0,97 | 1,08 | 12,16 | 12,16 |
| MCTP1 | 1,00 | 1,12 | 1,15 | 0,97 | 1,06 | 11,45 | 11,45 |
| GAS1 | 1,22 | 1,30 | 0,98 | 0,59 | 1,02 | 10,47 | 10,47 |
| BIRC3 | 0,96 | 1,04 | 1,28 | 0,79 | 1,02 | 10,40 | 10,40 |
| BQ186674 | 0,76 | 0,95 | 1,30 | 1,03 | 1,01 | 10,28 | 10,28 |
| MAL | 0,88 | 1,00 | 1,10 | 0,98 | 0,99 | 9,79 | 9,79 |
| UBXD3 | 1,23 | 1,12 | 0,88 | 0,74 | 0,99 | 9,78 | 9,78 |
| WNT2B | 0,95 | 0,96 | 0,97 | 1,01 | 0,97 | 9,42 | 9,42 |
| BM716045 | 1,06 | 1,02 | 0,98 | 0,82 | 0,97 | 9,36 | 9,36 |
| SFRP1 | 1,04 | 1,01 | 0,91 | 0,91 | 0,97 | 9,30 | 9,30 |
| PLEKHH2 | 1,12 | 0,92 | 0,86 | 0,84 | 0,93 | 8,61 | 8,61 |
| GNG11 | 1,10 | 1,15 | 0,81 | 0,53 | 0,90 | 7,88 | 7,88 |
| CDH16 | 1,27 | 1,15 | 0,71 | 0,44 | 0,89 | 7,84 | 7,84 |
| AKR1C1 | 1,00 | 1,10 | 0,82 | 0,66 | 0,89 | 7,84 | 7,84 |
| MGC42367 | 1,23 | 1,12 | 0,77 | 0,46 | 0,89 | 7,83 | 7,83 |
| SFRP1 | 0,93 | 0,92 | 0,86 | 0,86 | 0,89 | 7,82 | 7,82 |
| AQP1 | 0,90 | 0,87 | 0,90 | 0,90 | 0,89 | 7,82 | 7,82 |
| RAFTLIN | 0,83 | 0,94 | 1,01 | 0,76 | 0,89 | 7,69 | 7,69 |
| FAM111A | 0,90 | 0,82 | 0,86 | 0,93 | 0,88 | 7,53 | 7,53 |
| FAM111A | 0,86 | 0,88 | 0,92 | 0,83 | 0,87 | 7,46 | 7,46 |
| ADAMTS1 | 1,06 | 0,93 | 0,83 | 0,65 | 0,87 | 7,39 | 7,39 |
| FHOD3 | 0,93 | 0,91 | 0,82 | 0,77 | 0,86 | 7,20 | 7,20 |
| DUSP23 | 0,93 | 0,89 | 0,75 | 0,81 | 0,85 | 7,00 | 7,00 |
| ITGB8 | 0,87 | 0,88 | 0,89 | 0,74 | 0,85 | 7,00 | 7,00 |
| ADAMTS1 | 1,02 | 0,92 | 0,80 | 0,64 | 0,84 | 6,99 | 6,99 |
| THC2134488 | 0,97 | 0,89 | 0,78 | 0,73 | 0,84 | 6,92 | 6,92 |
| IL15 | 0,87 | 0,92 | 0,89 | 0,64 | 0,83 | 6,75 | 6,75 |
| PLEKHH2 | 1,01 | 0,83 | 0,68 | 0,78 | 0,83 | 6,69 | 6,69 |
| AKR1C1 | 0,90 | 1,01 | 0,76 | 0,62 | 0,82 | 6,61 | 6,61 |
| IGFBP3 | 0,66 | 0,99 | 0,90 | 0,69 | 0,81 | 6,45 | 6,45 |
| CYP1A1 | 0,83 | 0,78 | 0,72 | 0,82 | 0,79 | 6,14 | 6,14 |
| PHLDA1 | 0,75 | 0,72 | 0,76 | 0,83 | 0,77 | 5,86 | 5,86 |
| TLR3 | 0,68 | 0,75 | 1,07 | 0,55 | 0,76 | 5,79 | 5,79 |
| GPC3 | 0,86 | 0,81 | 0,64 | 0,72 | 0,76 | 5,74 | 5,74 |
| AHRR | 0,83 | 0,79 | 0,67 | 0,74 | 0,76 | 5,73 | 5,73 |
| CACNG6 | 0,96 | 0,96 | 0,58 | 0,54 | 0,76 | 5,72 | 5,72 |
| AQP1 | 0,76 | 0,72 | 0,75 | 0,75 | 0,75 | 5,58 | 5,58 |
| AKR1C3 | 0,79 | 0,93 | 0,66 | 0,59 | 0,74 | 5,53 | 5,53 |
| FSTL1 | 0,87 | 0,74 | 0,81 | 0,53 | 0,74 | 5,48 | 5,48 |
| AHR | 0,82 | 0,81 | 0,74 | 0,57 | 0,73 | 5,40 | 5,40 |
| C1orf88 | 1,05 | 0,91 | 0,49 | 0,48 | 0,73 | 5,39 | 5,39 |
| CDKN1C | 0,68 | 0,82 | 0,70 | 0,72 | 0,73 | 5,38 | 5,38 |
| A_32_P32463 | 0,79 | 0,76 | 0,65 | 0,72 | 0,73 | 5,37 | 5,37 |
| PCDH9 | 0,53 | 0,77 | 0,78 | 0,81 | 0,72 | 5,31 | 5,31 |
| NTN4 | 0,88 | 0,75 | 0,60 | 0,67 | 0,72 | 5,29 | 5,29 |
| MID1 | 0,60 | 0,74 | 0,93 | 0,61 | 0,72 | 5,28 | 5,28 |
| CSPG2 | 0,80 | 0,80 | 0,77 | 0,50 | 0,72 | 5,22 | 5,22 |
| AL133090 | 0,96 | 0,89 | 0,66 | 0,35 | 0,71 | 5,15 | 5,15 |
| DDC | 0,76 | 1,01 | 0,50 | 0,55 | 0,71 | 5,08 | 5,08 |
| AKR1C1 | 0,78 | 0,89 | 0,63 | 0,52 | 0,70 | 5,07 | 5,07 |
| PHLDA1 | 0,68 | 0,67 | 0,64 | 0,79 | 0,70 | 4,98 | 4,98 |
| KCNH2 | 0,38 | 0,83 | 0,90 | 0,65 | 0,69 | 4,86 | 4,86 |
| ITGB8 | 0,67 | 0,62 | 0,75 | 0,66 | 0,68 | 4,74 | 4,74 |
| ST6GAL1 | 0,78 | 0,91 | 0,69 | 0,32 | 0,67 | 4,72 | 4,72 |
| TMEFF1 | 0,75 | 0,79 | 0,64 | 0,51 | 0,67 | 4,70 | 4,70 |
| FBXL16 | 0,96 | 0,88 | 0,46 | 0,38 | 0,67 | 4,69 | 4,69 |
| ATP8A2 | 0,79 | 0,76 | 0,76 | 0,34 | 0,66 | 4,60 | 4,60 |
| CART1 | 0,69 | 0,76 | 0,78 | 0,40 | 0,66 | 4,57 | 4,57 |
| C9orf150 | 0,57 | 0,62 | 0,69 | 0,75 | 0,66 | 4,53 | 4,53 |
| PHLDA1 | 0,63 | 0,59 | 0,66 | 0,72 | 0,65 | 4,47 | 4,47 |
| HDAC9 | 0,77 | 0,55 | 0,71 | 0,55 | 0,64 | 4,41 | 4,41 |
| GLS | 0,65 | 0,62 | 0,74 | 0,56 | 0,64 | 4,39 | 4,39 |
| GATS | 0,79 | 0,73 | 0,55 | 0,46 | 0,63 | 4,30 | 4,30 |
| CNTNAP3 | 0,72 | 0,70 | 0,71 | 0,40 | 0,63 | 4,30 | 4,30 |
| PDE4B | 0,84 | 0,61 | 0,62 | 0,43 | 0,63 | 4,23 | 4,23 |
| DKFZp586I1420 | 0,63 | 0,66 | 0,65 | 0,56 | 0,62 | 4,20 | 4,20 |
| ZNRF2 | 0,64 | 0,60 | 0,66 | 0,58 | 0,62 | 4,18 | 4,18 |
| FGF2 | 0,73 | 0,79 | 0,57 | 0,39 | 0,62 | 4,17 | 4,17 |
| SP5 | 0,72 | 0,65 | 0,64 | 0,47 | 0,62 | 4,17 | 4,17 |
| LAMA2 | 0,46 | 0,63 | 0,77 | 0,59 | 0,61 | 4,08 | 4,08 |
| THC2056328 | 0,59 | 0,57 | 0,76 | 0,51 | 0,61 | 4,05 | 4,05 |
| KIAA1666 | 0,75 | 0,68 | 0,53 | 0,46 | 0,61 | 4,03 | 4,03 |
| A_23_P103951 | 0,73 | 0,67 | 0,61 | 0,41 | 0,60 | 4,02 | 4,02 |
| IL1R1 | 0,39 | 0,62 | 0,77 | 0,63 | 0,60 | 4,00 | 4,00 |
| CD55 | 0,74 | 0,66 | 0,59 | 0,39 | 0,59 | 3,93 | 3,93 |
| MANEAL | 0,75 | 0,70 | 0,55 | 0,37 | 0,59 | 3,93 | 3,93 |
| AK026140 | 0,68 | 0,61 | 0,52 | 0,57 | 0,59 | 3,93 | 3,93 |
| FXYD2 | 0,43 | 0,58 | 0,70 | 0,64 | 0,59 | 3,88 | 3,88 |
| CD40 | 0,60 | 0,65 | 0,56 | 0,54 | 0,59 | 3,87 | 3,87 |
| KIAA1505 | 0,76 | 0,65 | 0,62 | 0,31 | 0,59 | 3,86 | 3,86 |
| DEPDC6 | 0,65 | 0,59 | 0,68 | 0,42 | 0,59 | 3,85 | 3,85 |
| GLS | 0,58 | 0,54 | 0,71 | 0,52 | 0,59 | 3,85 | 3,85 |
| PPP2R2C | 0,45 | 0,51 | 0,69 | 0,68 | 0,58 | 3,82 | 3,82 |
| NRP1 | 0,44 | 0,60 | 0,70 | 0,58 | 0,58 | 3,80 | 3,80 |
| SP5 | 0,67 | 0,64 | 0,56 | 0,43 | 0,58 | 3,77 | 3,77 |
| ARHGDIB | 0,46 | 0,56 | 0,72 | 0,56 | 0,57 | 3,75 | 3,75 |
| RA12 | 0,84 | 0,64 | 0,34 | 0,47 | 0,57 | 3,74 | 3,74 |
| LOC284262 | 0,70 | 0,74 | 0,52 | 0,31 | 0,57 | 3,69 | 3,69 |
| TXNRD3 | 0,51 | 0,58 | 0,63 | 0,54 | 0,57 | 3,68 | 3,68 |
| HIVEP1 | 0,54 | 0,54 | 0,63 | 0,56 | 0,57 | 3,68 | 3,68 |
| BC042017 | 0,38 | 0,65 | 0,72 | 0,51 | 0,57 | 3,67 | 3,67 |
| ABCB2 | 0,56 | 0,58 | 0,64 | 0,48 | 0,56 | 3,66 | 3,66 |
| GLS | 0,48 | 0,63 | 0,65 | 0,48 | 0,56 | 3,65 | 3,65 |
| RASSF8 | 0,64 | 0,59 | 0,52 | 0,49 | 0,56 | 3,63 | 3,63 |
| CR622072 | 0,58 | 0,47 | 0,64 | 0,54 | 0,56 | 3,62 | 3,62 |
| LITAF | 0,68 | 0,59 | 0,50 | 0,45 | 0,55 | 3,56 | 3,56 |
| IGF2 | 0,67 | 0,67 | 0,49 | 0,38 | 0,55 | 3,56 | 3,56 |
| CYR61 | 0,61 | 0,49 | 0,51 | 0,59 | 0,55 | 3,55 | 3,55 |
| PHF15 | 0,55 | 0,55 | 0,56 | 0,54 | 0,55 | 3,54 | 3,54 |
| ProSAPiP1 | 0,63 | 0,51 | 0,51 | 0,54 | 0,55 | 3,51 | 3,51 |
| THC2227602 | 0,76 | 0,63 | 0,43 | 0,35 | 0,54 | 3,49 | 3,49 |
| LOC389722 | 0,61 | 0,59 | 0,53 | 0,43 | 0,54 | 3,46 | 3,46 |
| ANKRD18A | 0,66 | 0,53 | 0,58 | 0,35 | 0,53 | 3,38 | 3,38 |
| FBN1 | 0,71 | 0,54 | 0,55 | 0,31 | 0,53 | 3,37 | 3,37 |
| RPS6KA2 | 0,39 | 0,47 | 0,62 | 0,62 | 0,52 | 3,34 | 3,34 |
| GRB10 | 0,61 | 0,47 | 0,53 | 0,47 | 0,52 | 3,33 | 3,33 |
| AY336981 | 0,51 | 0,45 | 0,54 | 0,59 | 0,52 | 3,31 | 3,31 |
| RASSF8 | 0,61 | 0,56 | 0,49 | 0,42 | 0,52 | 3,31 | 3,31 |
| SLPI | 0,39 | 0,67 | 0,63 | 0,38 | 0,52 | 3,31 | 3,31 |
| SLPI | 0,37 | 0,69 | 0,64 | 0,37 | 0,52 | 3,30 | 3,30 |
| THC2095463 | 0,61 | 0,51 | 0,53 | 0,40 | 0,52 | 3,27 | 3,27 |
| COL16A1 | 0,57 | 0,63 | 0,50 | 0,35 | 0,51 | 3,26 | 3,26 |
| GRAMD3 | 0,55 | 0,52 | 0,55 | 0,43 | 0,51 | 3,26 | 3,26 |
| FXYD2 | 0,38 | 0,50 | 0,60 | 0,56 | 0,51 | 3,24 | 3,24 |
| PDGFB | 0,45 | 0,46 | 0,54 | 0,59 | 0,51 | 3,24 | 3,24 |
| FAM107B | 0,47 | 0,45 | 0,53 | 0,59 | 0,51 | 3,23 | 3,23 |
| LOC440934 | 0,41 | 0,58 | 0,65 | 0,40 | 0,51 | 3,22 | 3,22 |
| VCX | 0,37 | 0,54 | 0,56 | 0,56 | 0,51 | 3,21 | 3,21 |
| LAMB3 | 0,49 | 0,56 | 0,58 | 0,38 | 0,50 | 3,18 | 3,18 |
| CYR61 | 0,54 | 0,46 | 0,46 | 0,53 | 0,50 | 3,14 | 3,14 |
| NCALD | 0,60 | 0,50 | 0,42 | 0,47 | 0,50 | 3,14 | 3,14 |
| WNT5A | 0,58 | 0,41 | 0,47 | 0,52 | 0,49 | 3,12 | 3,12 |
| ABCC3 | 0,69 | 0,54 | 0,41 | 0,33 | 0,49 | 3,10 | 3,10 |
| GLIS1 | 0,61 | 0,50 | 0,45 | 0,40 | 0,49 | 3,09 | 3,09 |
| JAG1 | 0,60 | 0,50 | 0,45 | 0,39 | 0,49 | 3,06 | 3,06 |
| NRL | 0,48 | 0,51 | 0,52 | 0,41 | 0,48 | 3,03 | 3,03 |
| AGT | 0,43 | 0,38 | 0,42 | 0,68 | 0,48 | 3,01 | 3,01 |
| TMSB4X | 0,38 | 0,42 | 0,59 | 0,48 | 0,47 | 2,93 | 2,93 |
| CCPG1 | 0,53 | 0,49 | 0,44 | 0,38 | 0,46 | 2,90 | 2,90 |
| ADRA2C | 0,45 | 0,44 | 0,51 | 0,45 | 0,46 | 2,88 | 2,88 |
| BM665539 | 0,39 | 0,39 | 0,53 | 0,50 | 0,45 | 2,84 | 2,84 |
| TEX15 | 0,44 | 0,41 | 0,53 | 0,41 | 0,45 | 2,82 | 2,82 |
| SEMA3B | 0,42 | 0,44 | 0,48 | 0,45 | 0,45 | 2,81 | 2,81 |
| NYD-SP18 | 0,51 | 0,51 | 0,38 | 0,39 | 0,45 | 2,79 | 2,79 |
| ASNS | 0,50 | 0,37 | 0,48 | 0,43 | 0,44 | 2,79 | 2,79 |
| NFKBIZ | 0,43 | 0,31 | 0,54 | 0,49 | 0,44 | 2,77 | 2,77 |
| AK096677 | 0,53 | 0,38 | 0,49 | 0,37 | 0,44 | 2,76 | 2,76 |
| CA313037 | 0,52 | 0,50 | 0,41 | 0,33 | 0,44 | 2,76 | 2,76 |
| PTPRM | 0,45 | 0,43 | 0,45 | 0,42 | 0,44 | 2,75 | 2,75 |
| SLC1A1 | 0,52 | 0,44 | 0,36 | 0,43 | 0,44 | 2,74 | 2,74 |
| GRB10 | 0,40 | 0,35 | 0,50 | 0,50 | 0,44 | 2,73 | 2,73 |
| NQO1 | 0,46 | 0,41 | 0,45 | 0,42 | 0,44 | 2,73 | 2,73 |
| A_24_P401051 | 0,36 | 0,38 | 0,51 | 0,48 | 0,43 | 2,72 | 2,72 |
| GPR161 | 0,50 | 0,47 | 0,39 | 0,37 | 0,43 | 2,71 | 2,71 |
| A_32_P32905 | 0,58 | 0,41 | 0,43 | 0,30 | 0,43 | 2,70 | 2,70 |
| LOC389652 | 0,47 | 0,35 | 0,46 | 0,42 | 0,43 | 2,68 | 2,68 |
| SRGAP3 | 0,36 | 0,35 | 0,59 | 0,41 | 0,43 | 2,67 | 2,67 |
| PDE4D | 0,44 | 0,42 | 0,34 | 0,49 | 0,42 | 2,64 | 2,64 |
| THC2055165 | 0,49 | 0,40 | 0,43 | 0,33 | 0,41 | 2,58 | 2,58 |
| LOC63920 | 0,51 | 0,37 | 0,37 | 0,39 | 0,41 | 2,56 | 2,56 |
| AK022020 | 0,52 | 0,44 | 0,34 | 0,33 | 0,41 | 2,55 | 2,55 |
| MGAT4A | 0,41 | 0,38 | 0,37 | 0,44 | 0,40 | 2,53 | 2,53 |
| THC2201936 | 0,47 | 0,36 | 0,42 | 0,36 | 0,40 | 2,52 | 2,52 |
| THC2091303 | 0,47 | 0,48 | 0,33 | 0,32 | 0,40 | 2,51 | 2,51 |
| FCRL2 | 0,49 | 0,40 | 0,38 | 0,32 | 0,40 | 2,51 | 2,51 |
| PDE4DIP | 0,41 | 0,37 | 0,44 | 0,38 | 0,40 | 2,51 | 2,51 |
| WBP5 | 0,41 | 0,45 | 0,39 | 0,35 | 0,40 | 2,50 | 2,50 |
| PIM1 | 0,53 | 0,43 | 0,32 | 0,31 | 0,40 | 2,50 | 2,50 |
| NUPR1 | 0,45 | 0,34 | 0,43 | 0,37 | 0,40 | 2,49 | 2,49 |
| SMAD7 | 0,51 | 0,39 | 0,33 | 0,34 | 0,39 | 2,48 | 2,48 |
| LOC63920 | 0,47 | 0,37 | 0,36 | 0,37 | 0,39 | 2,47 | 2,47 |
| STAT1 | 0,45 | 0,49 | 0,30 | 0,33 | 0,39 | 2,47 | 2,47 |
| AK057151 | 0,51 | 0,35 | 0,36 | 0,34 | 0,39 | 2,46 | 2,46 |
| PPM1H | 0,46 | 0,39 | 0,35 | 0,36 | 0,39 | 2,45 | 2,45 |
| BM806490 | 0,40 | 0,39 | 0,40 | 0,36 | 0,39 | 2,45 | 2,45 |
| AL390181 | 0,44 | 0,40 | 0,37 | 0,31 | 0,38 | 2,41 | 2,41 |
| LOC150356 | 0,40 | 0,36 | 0,41 | 0,34 | 0,38 | 2,38 | 2,38 |
| WNT7B | 0,37 | 0,36 | 0,40 | 0,37 | 0,38 | 2,37 | 2,37 |
| BF210146 | 0,41 | 0,32 | 0,37 | 0,39 | 0,37 | 2,36 | 2,36 |
| LRP11 | 0,42 | 0,41 | 0,32 | 0,33 | 0,37 | 2,33 | 2,33 |
| FGFR2 | 0,37 | 0,38 | 0,33 | 0,33 | 0,36 | 2,27 | 2,27 |
| MT2A | 0,44 | 0,34 | 0,32 | 0,32 | 0,35 | 2,26 | 2,26 |
| BF378046 | 0,38 | 0,31 | 0,37 | 0,33 | 0,35 | 2,25 | 2,25 |
| MT2A | 0,42 | 0,33 | 0,32 | 0,32 | 0,35 | 2,22 | 2,22 |
| BC037328 | 0,38 | 0,32 | 0,37 | 0,31 | 0,35 | 2,21 | 2,21 |
| MT2A | 0,43 | 0,33 | 0,31 | 0,31 | 0,34 | 2,21 | 2,21 |
| ZNF323 | 0,33 | 0,35 | 0,34 | 0,35 | 0,34 | 2,20 | 2,20 |
| TBC1D8 | 0,41 | 0,31 | 0,31 | 0,33 | 0,34 | 2,18 | 2,18 |
| BLVRA | 0,34 | 0,34 | 0,31 | 0,35 | 0,33 | 2,16 | 2,16 |
| FGFR2 | 0,36 | 0,36 | 0,30 | 0,31 | 0,33 | 2,15 | 2,15 |

**Table 4 : List of the under-expressed genes by at least two fold in the docetaxel resistant cell-lines.**

| | **Log(Ratio) for each dose** | | | | **Log** **(ratio)** | **Ratio** | **Fold change** |
|---|---|---|---|---|---|---|---|
| Primary Sequence Name | **50nM** | **25nM** | **12nM** | **5nM** | **Mean** | **Mean** | **Mean** |
| PIK3C3 | -0,32 | -0,32 | -0,33 | -0,34 | -0,33 | 0,47 | -2,12 |
| SCARB1 | -0,39 | -0,32 | -0,35 | -0,35 | -0,35 | 0,45 | -2,24 |
| ASGR1 | -0,32 | -0,39 | -0,40 | -0,39 | -0,38 | 0,42 | -2,38 |
| FLJ22659 | -0,42 | -0,31 | -0,37 | -0,40 | -0,38 | 0,42 | -2,38 |
| ASMTL | -0,32 | -0,39 | -0,46 | -0,35 | -0,38 | 0,42 | -2,40 |
| ARHGAP10 | -0,46 | -0,37 | -0,31 | -0,39 | -0,38 | 0,41 | -2,41 |
| EDG6 | -0,44 | -0,43 | -0,36 | -0,34 | -0,39 | 0,41 | -2,45 |
| KCNC3 | -0,46 | -0,43 | -0,32 | -0,35 | -0,39 | 0,41 | -2,45 |
| MGC11332 | -0,48 | -0,39 | -0,36 | -0,34 | -0,39 | 0,41 | -2,47 |
| NRG1 | -0,50 | -0,39 | -0,37 | -0,32 | -0,39 | 0,40 | -2,47 |
| LOC339240 | -0,41 | -0,45 | -0,30 | -0,42 | -0,39 | 0,40 | -2,48 |
| PTPN3 | -0,43 | -0,36 | -0,36 | -0,43 | -0,39 | 0,40 | -2,48 |
| AK123483 | -0,39 | -0,38 | -0,42 | -0,41 | -0,40 | 0,40 | -2,51 |
| NRG1 | -0,52 | -0,40 | -0,37 | -0,32 | -0,40 | 0,40 | -2,51 |
| FAM80A | -0,33 | -0,31 | -0,46 | -0,52 | -0,41 | 0,39 | -2,55 |
| BAMBI | -0,55 | -0,36 | -0,32 | -0,40 | -0,41 | 0,39 | -2,56 |
| PTPN3 | -0,49 | -0,42 | -0,33 | -0,43 | -0,42 | 0,38 | -2,62 |
| SAMD8 | -0,56 | -0,44 | -0,32 | -0,36 | -0,42 | 0,38 | -2,62 |
| KCNMB4 | -0,58 | -0,39 | -0,37 | -0,36 | -0,43 | 0,38 | -2,67 |
| SPG20 | -0,53 | -0,45 | -0,35 | -0,40 | -0,43 | 0,37 | -2,70 |
| RGS16 | -0,58 | -0,43 | -0,34 | -0,38 | -0,43 | 0,37 | -2,71 |
| UGT2B7 | -0,65 | -0,45 | -0,31 | -0,34 | -0,44 | 0,37 | -2,74 |
| LMBRD2 | -0,39 | -0,42 | -0,48 | -0,48 | -0,44 | 0,36 | -2,77 |
| TMPRSS4 | -0,68 | -0,39 | -0,35 | -0,37 | -0,45 | 0,36 | -2,79 |
| PDK1 | -0,46 | -0,46 | -0,48 | -0,39 | -0,45 | 0,36 | -2,80 |
| RAB39B | -0,34 | -0,54 | -0,42 | -0,50 | -0,45 | 0,36 | -2,81 |
| HSPH1 | -0,68 | -0,50 | -0,31 | -0,35 | -0,46 | 0,35 | -2,89 |
| PSCDBP | -0,44 | -0,46 | -0,50 | -0,46 | -0,46 | 0,34 | -2,91 |
| UGT2B11 | -0,69 | -0,45 | -0,34 | -0,38 | -0,46 | 0,34 | -2,91 |
| ZNF516 | -0,55 | -0,46 | -0,39 | -0,49 | -0,47 | 0,34 | -2,95 |
| CKB | -0,58 | -0,49 | -0,36 | -0,46 | -0,47 | 0,34 | -2,98 |
| SLC7A8 | -0,34 | -0,42 | -0,61 | -0,56 | -0,48 | 0,33 | -3,05 |
| UGT2B28 | -0,76 | -0,50 | -0,34 | -0,37 | -0,49 | 0,32 | -3,10 |
| SEMA3C | -0,59 | -0,56 | -0,35 | -0,47 | -0,49 | 0,32 | -3,11 |
| PDK1 | -0,52 | -0,50 | -0,50 | -0,45 | -0,49 | 0,32 | -3,11 |
| GATA2 | -0,55 | -0,50 | -0,50 | -0,44 | -0,50 | 0,32 | -3,13 |
| THC2064535 | -0,52 | -0,45 | -0,51 | -0,51 | -0,50 | 0,32 | -3,16 |
| PDLIM5 | -0,60 | -0,53 | -0,42 | -0,45 | -0,50 | 0,32 | -3,16 |
| BX538293 | -0,54 | -0,56 | -0,39 | -0,52 | -0,50 | 0,31 | -3,18 |
| HYAL1 | -0,70 | -0,56 | -0,36 | -0,40 | -0,50 | 0,31 | -3,20 |
| UGT2B4 | -0,74 | -0,55 | -0,34 | -0,39 | -0,51 | 0,31 | -3,20 |
| THC2185385 | -0,54 | -0,48 | -0,39 | -0,62 | -0,51 | 0,31 | -3,21 |
| FLJ10159 | -0,56 | -0,49 | -0,46 | -0,53 | -0,51 | 0,31 | -3,24 |
| UGT2B10 | -0,75 | -0,54 | -0,35 | -0,41 | -0,51 | 0,31 | -3,26 |
| PRTFDC1 | -0,49 | -0,50 | -0,64 | -0,42 | -0,51 | 0,31 | -3,26 |
| A_24_P575267 | -0,78 | -0,50 | -0,36 | -0,42 | -0,51 | 0,31 | -3,26 |
| PTGES | -0,68 | -0,59 | -0,40 | -0,38 | -0,51 | 0,31 | -3,27 |
| DNAJC15 | -0,78 | -0,57 | -0,35 | -0,37 | -0,52 | 0,30 | -3,29 |
| NPAS1 | -0,67 | -0,57 | -0,46 | -0,37 | -0,52 | 0,30 | -3,29 |
| A_24_P478940 | -0,70 | -0,58 | -0,40 | -0,39 | -0,52 | 0,30 | -3,30 |
| FRMD4B | -0,49 | -0,50 | -0,58 | -0,52 | -0,52 | 0,30 | -3,34 |
| SLC7A11 | -0,58 | -0,62 | -0,39 | -0,52 | -0,53 | 0,30 | -3,36 |
| AF132203 | -0,61 | -0,47 | -0,42 | -0,61 | -0,53 | 0,30 | -3,36 |
| FLJ43855 | -0,61 | -0,54 | -0,50 | -0,46 | -0,53 | 0,30 | -3,38 |
| TFDP2 | -0,67 | -0,65 | -0,46 | -0,33 | -0,53 | 0,30 | -3,38 |
| PFKFB4 | -0,54 | -0,56 | -0,51 | -0,50 | -0,53 | 0,30 | -3,38 |
| TAC3 | -0,55 | -0,46 | -0,50 | -0,61 | -0,53 | 0,30 | -3,38 |
| ENC1 | -0,74 | -0,50 | -0,49 | -0,40 | -0,53 | 0,29 | -3,40 |
| EPAS1 | -0,54 | -0,50 | -0,49 | -0,61 | -0,54 | 0,29 | -3,43 |
| NRP2 | -0,72 | -0,54 | -0,49 | -0,42 | -0,54 | 0,29 | -3,48 |
| MFHAS1 | -0,56 | -0,56 | -0,53 | -0,53 | -0,54 | 0,29 | -3,50 |
| AK024680 | -0,68 | -0,50 | -0,47 | -0,52 | -0,54 | 0,29 | -3,51 |
| SYTL3 | -0,64 | -0,68 | -0,45 | -0,42 | -0,55 | 0,28 | -3,53 |
| BDNF | -0,63 | -0,46 | -0,50 | -0,62 | -0,55 | 0,28 | -3,57 |
| AL137342 | -0,80 | -0,41 | -0,41 | -0,64 | -0,56 | 0,27 | -3,67 |
| D4S234E | -0,69 | -0,44 | -0,46 | -0,68 | -0,57 | 0,27 | -3,70 |
| LCP1 | -0,92 | -0,52 | -0,30 | -0,54 | -0,57 | 0,27 | -3,72 |
| A_32_P95067 | -0,72 | -0,63 | -0,54 | -0,39 | -0,57 | 0,27 | -3,73 |
| THC2038567 | -0,86 | -0,75 | -0,38 | -0,32 | -0,57 | 0,27 | -3,75 |
| LMO1 | -0,61 | -0,56 | -0,61 | -0,52 | -0,58 | 0,27 | -3,77 |
| BSPRY | -0,72 | -0,46 | -0,52 | -0,61 | -0,58 | 0,26 | -3,78 |
| BDNF | -0,61 | -0,49 | -0,56 | -0,67 | -0,58 | 0,26 | -3,81 |
| UGT8 | -0,78 | -0,44 | -0,48 | -0,63 | -0,58 | 0,26 | -3,81 |
| LIPG | -0,85 | -0,67 | -0,33 | -0,51 | -0,59 | 0,26 | -3,89 |
| AW205591 | -0,74 | -0,79 | -0,52 | -0,32 | -0,59 | 0,26 | -3,90 |
| AKAP12 | -0,94 | -0,68 | -0,32 | -0,43 | -0,59 | 0,25 | -3,92 |
| B3GALT1 | -0,54 | -0,53 | -0,63 | -0,67 | -0,59 | 0,25 | -3,93 |
| CHST7 | -0,79 | -0,64 | -0,47 | -0,49 | -0,59 | 0,25 | -3,93 |
| NMNAT2 | -0,86 | -0,74 | -0,44 | -0,36 | -0,60 | 0,25 | -3,96 |
| SLC12A3 | -0,86 | -0,67 | -0,31 | -0,57 | -0,60 | 0,25 | -3,98 |
| SLC22A2 | -0,94 | -0,67 | -0,40 | -0,43 | -0,61 | 0,25 | -4,05 |
| LMBRD2 | -0,67 | -0,61 | -0,55 | -0,62 | -0,61 | 0,24 | -4,09 |
| SCD | -0,68 | -0,51 | -0,53 | -0,74 | -0,61 | 0,24 | -4,11 |
| AMPH | -0,89 | -0,57 | -0,49 | -0,53 | -0,62 | 0,24 | -4,17 |
| ANKRD37 | -0,76 | -0,66 | -0,48 | -0,59 | -0,62 | 0,24 | -4,18 |
| LIN7A | -0,98 | -0,59 | -0,39 | -0,52 | -0,62 | 0,24 | -4,19 |
| PHEX | -0,88 | -0,76 | -0,42 | -0,44 | -0,62 | 0,24 | -4,22 |
| C1QL1 | -0,59 | -0,55 | -0,72 | -0,64 | -0,63 | 0,24 | -4,24 |
| EPAS1 | -0,63 | -0,57 | -0,63 | -0,72 | -0,64 | 0,23 | -4,34 |
| KCNC4 | -0,75 | -0,67 | -0,50 | -0,64 | -0,64 | 0,23 | -4,36 |
| FOXL2 | -0,81 | -0,68 | -0,47 | -0,61 | -0,64 | 0,23 | -4,38 |
| SCD | -0,77 | -0,58 | -0,62 | -0,61 | -0,64 | 0,23 | -4,40 |
| FGFBP1 | -0,99 | -0,64 | -0,47 | -0,50 | -0,65 | 0,22 | -4,48 |
| CASP8 | -0,40 | -0,77 | -0,65 | -0,80 | -0,65 | 0,22 | -4,51 |
| LZTS1 | -0,81 | -0,71 | -0,65 | -0,46 | -0,66 | 0,22 | -4,53 |
| SYTL3 | -0,74 | -0,81 | -0,54 | -0,53 | -0,66 | 0,22 | -4,54 |
| HSHPX5 | -0,80 | -0,61 | -0,63 | -0,59 | -0,66 | 0,22 | -4,56 |
| NLGN1 | -0,41 | -0,33 | -0,86 | -1,05 | -0,66 | 0,22 | -4,62 |
| MGST1 | -0,98 | -0,68 | -0,45 | -0,58 | -0,67 | 0,21 | -4,70 |
| PLXNA2 | -0,73 | -0,60 | -0,49 | -0,88 | -0,68 | 0,21 | -4,75 |
| ST8SIA4 | -0,75 | -0,53 | -0,59 | -0,85 | -0,68 | 0,21 | -4,77 |
| THC2050576 | -1,00 | -0,72 | -0,50 | -0,51 | -0,68 | 0,21 | -4,78 |
| SLC3A1 | -0,87 | -0,74 | -0,62 | -0,51 | -0,68 | 0,21 | -4,82 |
| TNRC9 | -0,82 | -0,62 | -0,51 | -0,79 | -0,68 | 0,21 | -4,84 |
| AK022997 | -1,23 | -0,47 | -0,36 | -0,70 | -0,69 | 0,20 | -4,88 |
| UGT8 | -0,94 | -0,51 | -0,56 | -0,76 | -0,69 | 0,20 | -4,93 |
| LETM2 | -1,04 | -0,83 | -0,41 | -0,50 | -0,70 | 0,20 | -4,97 |
| PLAC8 | -1,07 | -0,80 | -0,39 | -0,53 | -0,70 | 0,20 | -4,99 |
| DIAPH2 | -1,18 | -0,71 | -0,38 | -0,54 | -0,70 | 0,20 | -5,05 |
| BC014452 | -0,91 | -0,61 | -0,56 | -0,76 | -0,71 | 0,20 | -5,12 |
| SUNC1 | -0,95 | -0,79 | -0,60 | -0,52 | -0,72 | 0,19 | -5,20 |
| DUSP13 | -0,84 | -0,74 | -0,61 | -0,71 | -0,73 | 0,19 | -5,32 |
| AUTS2 | -0,59 | -0,52 | -0,84 | -1,02 | -0,74 | 0,18 | -5,51 |
| PLAC8 | -1,15 | -0,84 | -0,43 | -0,55 | -0,74 | 0,18 | -5,52 |
| THC2210862 | -1,43 | -0,77 | -0,43 | -0,36 | -0,75 | 0,18 | -5,61 |
| MSX2 | -0,96 | -0,69 | -0,72 | -0,68 | -0,76 | 0,17 | -5,79 |
| SMAD9 | -0,79 | -0,80 | -0,77 | -0,70 | -0,76 | 0,17 | -5,81 |
| TTN | -0,86 | -0,74 | -0,55 | -0,91 | -0,77 | 0,17 | -5,82 |
| LRRN6C | -1,00 | -0,73 | -0,53 | -0,82 | -0,77 | 0,17 | -5,88 |
| MEIS2 | -1,12 | -0,92 | -0,67 | -0,38 | -0,77 | 0,17 | -5,91 |
| DHRS3 | -1,03 | -0,75 | -0,65 | -0,67 | -0,77 | 0,17 | -5,95 |
| OLR1 | -1,34 | -0,95 | -0,31 | -0,58 | -0,80 | 0,16 | -6,25 |
| NSBP1 | -1,18 | -0,84 | -0,55 | -0,64 | -0,80 | 0,16 | -6,30 |
| MOXD1 | -0,90 | -0,70 | -0,80 | -0,82 | -0,80 | 0,16 | -6,37 |
| DCAMKL1 | -1,11 | -0,84 | -0,65 | -0,63 | -0,81 | 0,16 | -6,40 |
| C12orf59 | -1,01 | -0,81 | -0,64 | -0,76 | -0,81 | 0,16 | -6,42 |
| A_23_P136857 | -1,10 | -0,99 | -0,71 | -0,52 | -0,83 | 0,15 | -6,75 |
| BF675806 | -1,09 | -0,87 | -0,72 | -0,65 | -0,83 | 0,15 | -6,82 |
| SLC44A5 | -1,28 | -0,83 | -0,73 | -0,54 | -0,84 | 0,14 | -6,96 |
| SALL1 | -1,26 | -1,04 | -0,74 | -0,36 | -0,85 | 0,14 | -7,03 |
| GPR177 | -1,27 | -0,89 | -0,62 | -0,63 | -0,85 | 0,14 | -7,13 |
| AK3L1 | -1,27 | -0,99 | -0,70 | -0,49 | -0,86 | 0,14 | -7,28 |
| AK3L1 | -1,14 | -0,99 | -0,76 | -0,56 | -0,86 | 0,14 | -7,29 |
| FZD8 | -0,94 | -0,79 | -0,85 | -0,93 | -0,88 | 0,13 | -7,52 |
| THC2088463 | -0,98 | -0,95 | -0,73 | -0,85 | -0,88 | 0,13 | -7,55 |
| FLJ39502 | -0,87 | -0,97 | -0,69 | -1,01 | -0,88 | 0,13 | -7,65 |
| PROS1 | -1,02 | -0,87 | -0,69 | -0,97 | -0,89 | 0,13 | -7,76 |
| PTPRD | -1,28 | -0,82 | -0,66 | -0,85 | -0,90 | 0,13 | -7,97 |
| PDE1A | -1,24 | -1,13 | -0,72 | -0,53 | -0,90 | 0,12 | -8,01 |
| MYB | -0,89 | -0,81 | -0,86 | -1,05 | -0,90 | 0,12 | -8,01 |
| SLC16A10 | -1,19 | -1,22 | -0,74 | -0,49 | -0,91 | 0,12 | -8,08 |
| GJA7 | -1,18 | -0,87 | -0,80 | -0,80 | -0,91 | 0,12 | -8,12 |
| GAL | -1,22 | -0,96 | -0,71 | -0,81 | -0,92 | 0,12 | -8,40 |
| CPM | -1,17 | -0,95 | -0,83 | -0,79 | -0,93 | 0,12 | -8,57 |
| PDE1A | -1,40 | -1,12 | -0,73 | -0,52 | -0,94 | 0,11 | -8,75 |
| PLXNA2 | -1,10 | -0,82 | -0,58 | -1,28 | -0,95 | 0,11 | -8,86 |
| PDE1A | -1,21 | -1,30 | -0,76 | -0,53 | -0,95 | 0,11 | -8,88 |
| AW467174 | -1,37 | -1,23 | -0,73 | -0,49 | -0,95 | 0,11 | -8,95 |
| PLAT | -1,34 | -1,10 | -0,43 | -0,95 | -0,95 | 0,11 | -8,98 |
| LOC441047 | -1,28 | -1,19 | -0,77 | -0,58 | -0,95 | 0,11 | -9,01 |
| CXCR4 | -1,59 | -1,32 | -0,53 | -0,41 | -0,96 | 0,11 | -9,16 |
| AK3L1 | -1,35 | -1,13 | -0,82 | -0,57 | -0,97 | 0,11 | -9,27 |
| SMPDL3A | -1,46 | -1,05 | -0,67 | -0,71 | -0,97 | 0,11 | -9,37 |
| KIAA0960 | -1,36 | -0,63 | -0,52 | -1,38 | -0,97 | 0,11 | -9,39 |
| LHFP | -1,26 | -1,02 | -0,97 | -0,66 | -0,98 | 0,11 | -9,52 |
| CPM | -1,17 | -0,96 | -0,92 | -0,86 | -0,98 | 0,11 | -9,52 |
| A_24_P345290 | -1,33 | -1,22 | -0,85 | -0,55 | -0,99 | 0,10 | -9,75 |
| RXFP1 | -1,56 | -1,09 | -0,69 | -0,64 | -1,00 | 0,10 | -9,94 |
| PNOC | -1,04 | -1,15 | -0,92 | -0,91 | -1,01 | 0,10 | -10,18 |
| GALNT14 | -1,39 | -1,16 | -0,68 | -0,81 | -1,01 | 0,10 | -10,26 |
| TM4SF1 | -0,98 | -1,11 | -1,03 | -0,98 | -1,02 | 0,09 | -10,59 |
| ZAR1 | -1,08 | -0,98 | -1,03 | -1,04 | -1,03 | 0,09 | -10,75 |
| A_23_P10091 | -1,12 | -0,86 | -1,33 | -0,91 | -1,05 | 0,09 | -11,31 |
| GLT8D2 | -1,28 | -1,21 | -0,92 | -0,84 | -1,06 | 0,09 | -11,53 |
| RXFP1 | -1,81 | -1,24 | -0,71 | -0,55 | -1,08 | 0,08 | -11,90 |
| CGNL1 | -1,40 | -1,26 | -0,95 | -0,73 | -1,08 | 0,08 | -12,08 |
| AK094972 | -1,43 | -1,35 | -0,75 | -1,05 | -1,14 | 0,07 | -13,93 |
| LRCH2 | -1,27 | -1,00 | -1,15 | -1,34 | -1,19 | 0,06 | -15,54 |
| BM930757 | -1,30 | -1,11 | -1,20 | -1,20 | -1,20 | 0,06 | -15,91 |
| ATP8A1 | -1,34 | -1,38 | -1,15 | -0,98 | -1,21 | 0,06 | -16,34 |
| SOX9 | -1,25 | -1,31 | -1,34 | -0,97 | -1,22 | 0,06 | -16,47 |
| SLC39A8 | -1,36 | -1,12 | -1,32 | -1,22 | -1,25 | 0,06 | -17,94 |
| TMEM47 | -1,59 | -1,10 | -1,13 | -1,29 | -1,28 | 0,05 | -18,94 |
| SLC10A4 | -1,23 | -1,12 | -1,28 | -1,51 | -1,29 | 0,05 | -19,33 |
| SLC1A3 | -1,49 | -1,34 | -1,01 | -1,38 | -1,30 | 0,05 | -20,08 |
| EDG7 | -1,35 | -1,32 | -1,27 | -1,36 | -1,33 | 0,05 | -21,23 |
| ITGA2 | -1,74 | -1,39 | -1,03 | -1,45 | -1,40 | 0,04 | -25,31 |
| SLC1A3 | -1,75 | -1,42 | -1,09 | -1,37 | -1,41 | 0,04 | -25,49 |
| PLCXD3 | -1,28 | -1,22 | -1,33 | -1,93 | -1,44 | 0,04 | -27,49 |
| BF514799 | -1,37 | -1,45 | -1,38 | -1,70 | -1,47 | 0,03 | -29,80 |
| SLC16A12 | -1,56 | -1,34 | -1,54 | -1,50 | -1,48 | 0,03 | -30,49 |
| THC2208430 | -1,88 | -1,83 | -1,03 | -1,39 | -1,53 | 0,03 | -34,08 |
| THC2182743 | -1,60 | -1,49 | -1,47 | -1,67 | -1,56 | 0,03 | -36,19 |
| C4orf18 | -1,67 | -1,49 | -1,58 | -1,57 | -1,58 | 0,03 | -37,64 |
| ANKRD38 | -1,71 | -1,50 | -1,53 | -1,62 | -1,59 | 0,03 | -38,90 |
| CALCRL | -1,74 | -1,77 | -1,92 | -1,84 | -1,82 | 0,02 | -66,13 |
| LOC152573 | -2,17 | -2,80 | -2,05 | -1,79 | -2,20 | 0,01 | -159,40 |

## Claims

1. An *in vitro* method for predicting or monitoring whether a patient affected by a cancer is responsive to a treatment with a molecule of the taxoid family, wherein the method comprises:
1) providing a biological sample from said subject; 2) determining in the biological sample the expression level of at least 5 genes selected from the group consisting of the genes listed in Tables 1 and 2, thereby predicting or monitoring whether a patient affected by a prostate cancer is responsive to a treatment with a molecule of the taxoid family.

2. The method according to claim 1, wherein the method further comprises comparing the expression level of said at least 5 genes to a reference expression level, the reference expression level being the expression level of the genes in cell-lines or patients sensitive to the treatment by the molecule of the taxoid family.

3. The method according to claim 2, wherein the over-expression of genes from Table 1 and/or the under-expression of genes from Table 2 are indicative of a resistance to the treatment by the molecule of the taxoid family.

4. The method according to anyone of claims 1-3, wherein the at least 5 genes are selected from one of the following groups or a combination thereof:
a) RPIB9, CXCL2, AL137761, TFPI2, THC2051204, TNF, ABCB1, PURG, ADAMTS5, MCTP1, SPTLC2L, OAS3, MCTP1, GAS1, BIRC3, BQ186674, MAL, UBXD3, WNT2B, GALNT14, TM4SF1, ZAR1, A_23_P10091, GLT8D2, RXFP1, CGNL1, AK094972, LRCH2, BM930757, ATP8A1, SOX9, SLC39A8, TMEM47, SLC10A4, SLC1A3, EDG7, ITGA2, PLCXD3, BF514799, SLC16A12, THC2208430, THC2182743, C4orf18, ANKRD38, CALCRL, and LOC152573, preferably RPIB9, CXCL2, TFPI2, TNF, ABCB1, ADAMTS5, PURG, OAS3, GAS1, BIRC3, MAL, GALNT14, TM4SF1, RXFP1, ATP8A1, SOX9, SLC39A8, EDG7, ITGA2, SLC1A3, CALCRL and LOC152573;
b) RPIB9, TFPI2, ABCB1, BIRC3, WNT2B, SFRP1, FSTL1, AHR, CDKN1C, ABCB2, CYR61, WNT5A, ABCC3, JAG1, STAT1, WNT7B, CASP8, LZTS1, FZD8, GALNT14, RXFP1 and LOC152573;
c) ABCC3, CD55, COL16A1, DHRS3, FSTL1, GLS, HDL, HIVE1, LAMA2, LAMB3, LIPG, LITAF, MAL, MFHAS1, NFKBIZ, NRP1, NRP2, OAS3, OLR1, PSCDBP, RFTN1, SCARAB1, SEMA3B, SEMA3C, SFRP1, SLC1A3, ST6GAL1, TLR3, TM4SF1 and TNF;
d) ADAMTS1, ADRA2C, AKAP12, CDKN1C, CYR61, FBN1, GAS1, GPC3, IGF2, IGFBP3, JAG1, MGST1, NTN4, PDE1A, PDE4B, PDE4D, PDE4DIP, PDGFB, PHLDA1, PIM1, PPP2R2C, RGS16, SCD, SLC1A1, SMPDL3A, TFP12 and VCAN;
e) ABCB1, AHR, AHRR, AMPH, BIRC3, CXCL2, CYP1A1, IL1R1, NQO1, PLAT, PLXNA2, SLC16A10, SLC3A1, SLC7A8, SLPI, TAP1, UGT8, UGT2B4, UGT2B7, UGT2B10, UGT2B11 and UGT2B28;
f) AQP1, ARHGDIB, BAMBI, CREB5, CXCR4, EPAS1, FGF2, FGFBP1, GRB10, IL15, MT2A, NUPR1, PDK1, PROS1, PTPN3, RPS6KA2, TFDP2, WNT2B, WNT5A and WNT7B;
g) AGT, ATP8A2, BDNF, EDG6, GAL, GATA2, ITGA2, LRP11, LZTS1, MYB, NCALD, PNOC, PTGES, SRGAP3, TAC3 and TTN;
h) AFF1, ASGR1, BLVRA, CASP8, CD40, KCNH2, NRG1, NRL, PHEX, PLAC8, SMAD7, SMAD9, SOX9, SPG20 and STAT1;
i) TNF, ABCB1, CYP1A1, AHRR, AHR, PP2R2C, ABCC3, NQO1, PIK3C3, UGT2B7, UGT2B11, UGT2B28, UGT2B4, UGT2B10, CHST7, MGST1 and UGT8; and,
j) Wnt2B, Wnt5A, Wnt7B, SFRP1, FSTL1, Jag1, Cyr61, LOC152573, FZD8 and FOXL2.

5. The method according to anyone of claims 1-4, wherein the molecule of the taxoid family is docetaxel, larotaxel, XRP6258, BMS-184476, BMS-188797, BMS-275183, ortataxel, RPR 109881A, RPR 116258, NBT-287, PG-paclitaxel, ABRAXANE®, Tesetaxel, IDN 5390, Taxoprexin, DHA-paclitaxel, and MAC-321, more preferably docetaxel.

6. The method according to anyone of claims 1-5, wherein the method comprises determining the expression level of at least 10, 15, 20, 25, 30, 40, 50, 60, 70, 80, 90, 100, 150, 200 or 300 genes from those listed in Tables 1 and 2.

7. The method according to anyone of claims 1-6, wherein the expression level of genes is determined by the quantity of protein or mRNA encoded by said genes.

8. The method according to anyone of claims 1-7, wherein the biological sample is a cancer sample.

9. The method according to anyone of claims 1-8, wherein the cancer is selected from the group consisting of the breast cancer, the lung cancer, the prostate cancer, the gastric cancer and the head and neck cancer, more preferably a prostate cancer.

10. A kit for predicting or monitoring whether a patient affected by a cancer is responsive to a treatment with a molecule of the taxoid family, wherein the kit comprises detection means selected from the group consisting of a pair of primers, a probe and an antibody specific to at least 5 genes selected from the group consisting of the genes listed in Tables 1 and 2.

11. A DNA chip comprising a solid support which carries nucleic acids that are specific to at least 5 genes selected from the group consisting of the genes listed in Tables 1 and 2.

12. Kit according to claim 10 or DNA chip according to claim 11, wherein the at least 5 genes are selected from one of the following groups or a combination thereof:
a) RPIB9, CXCL2, AL137761, TFPI2, THC2051204, TNF, ABCB1, PURG, ADAMTS5, MCTP1, SPTLC2L, OAS3, MCTP1, GAS1, BIRC3, BQ186674, MAL, UBXD3, WNT2B, GALNT14, TM4SF1, ZAR1, A_23_P10091, GLT8D2, RXFP1, CGNL1, AK094972, LRCH2, BM930757, ATP8A1, SOX9, SLC39A8, TMEM47, SLC10A4, SLC1A3, EDG7, ITGA2, PLCXD3, BF514799, SLC16A12, THC2208430, THC2182743, C4orfl8, ANKRD38, CALCRL, and LOC152573, preferably RPIB9, CXCL2, TFPI2, TNF, ABCB1, ADAMTS5, PURG, OAS3, GAS1, BIRC3, MAL, GALNT14, TM4SF1, RXFP1, ATP8A1, SOX9, SLC39A8, EDG7, ITGA2, SLC1A3, CALCRL and LOC152573;
b) RPIB9, TFPI2, ABCB1, BIRC3, WNT2B, SFRP1, FSTL1, AHR, CDKN1C, ABCB2, CYR61, WNT5A, ABCC3, JAG1, STAT1, WNT7B, CASP8, LZTS1, FZD8, GALNT14, RXFP1 and LOC152573;
c) ABCC3, CD55, COL16A1, DHRS3, FSTL1, GLS, HDL, HIVE1, LAMA2, LAMB3, LIPG, LITAF, MAL, MFHAS1, NFKBIZ, NRP1, NRP2, OAS3, OLR1, PSCDBP, RFTN1, SCARAB1, SEMA3B, SEMA3C, SFRP1, SLC1A3, ST6GAL1, TLR3, TM4SF1 and TNF;
d) ADAMTS1, ADRA2C, AKAP12, CDKN1C, CYR61, FBN1, GAS1, GPC3, IGF2, IGFBP3, JAG1, MGST1, NTN4, PDE1A, PDE4B, PDE4D, PDE4DIP, PDGFB, PHLDA1, PIM1, PPP2R2C, RGS16, SCD, SLC1A1, SMPDL3A, TFPI2 and VCAN;
e) ABCB1, AHR, AHRR, AMPH, BIRC3, CXCL2, CYP1A1, IL1R1, NQO1, PLAT, PLXNA2, SLC16A10, SLC3A1, SLC7A8, SLPI, TAP1, UGT8, UGT2B4, UGT2B7, UGT2B10, UGT2B11 and UGT2B28;
f) AQP1, ARHGDIB, BAMBI, CREB5, CXCR4, EPAS1, FGF2, FGFBP1, GRB10, IL15, MT2A, NUPR1, PDK1, PROS1, PTPN3, RPS6KA2, TFDP2, WNT2B, WNT5A and WNT7B;
g) AGT, ATP8A2, BDNF, EDG6, GAL, GATA2, ITGA2, LRP11, LZTS1, MYB, NCALD, PNOC, PTGES, SRGAP3, TAC3 and TTN;
h) AFF1, ASGR1, BLVRA, CASP8, CD40, KCNH2, NRG1, NRL, PHEX, PLAC8, SMAD7, SMAD9, SOX9, SPG20 and STAT1;
i) TNF, ABCB1, CYP1A1, AHRR, AHR, PP2R2C, ABCC3, NQO1, PIK3C3, UGT2B7, UGT2B11, UGT2B28, UGT2B4, UGT2B10, CHST7, MGST1 and UGT8; and,
j) Wnt2B, Wnt5A, Wnt7B, SFRP1, FSTL1, Jag1, Cyr61, LOC152573, FZD8 and FOXL2.

13. A method for screening or identifying a compound suitable for improving the treatment of a cancer with a molecule of the taxoid family or for reducing the resistance development during the treatment of a cancer with a molecule of the taxoid family, comprising
1) providing a cell-line with at least 5 genes over-expressed and/or under-expressed respectively selected from the group of over-expressed genes of Table 1 and under-expressed genes of Table 2; 2) contacting said cell-line with a test compound; 3) determining the expression level of said at least 5 genes; and, 4) selecting the compound which decreases the expression level of the over-expressed genes and increases the expression level of the under-expressed genes.

14. A method for screening or identifying a compound suitable for improving the treatment of a cancer with a molecule of the taxoid family or for reducing the resistance development during the treatment of a cancer with the molecule of the taxoid family, comprising
1) providing a cell-line sensitive to the molecule of the taxoid family; 2) contacting said cell-line with a test compound and the molecule of the taxoid family; 3) determining the expression level of said at least 5 genes selected from the genes listed in Tables 1 and 2; and, 4) selecting the compound which inhibits the appearance of an over-expression and/or an under-expression of at least 5 genes respectively selected from the group of genes of Table 1 and genes of Table 2.

15. A method for screening or identifying a compound suitable for improving the treatment of a cancer with a molecule of the taxoid family or for reducing the resistance development during the treatment of a cancer with the molecule of the taxoid family, comprising
1) providing a cell-line with at least on gene over-expressed and/or under-expressed respectively selected from the group consisting of RPIB9, CXCL2, AL137761, TFPI2, THC2051204, TNF, ABCB1, PURG, ADAMTS5, MCTP1, SPTLC2L, OAS3, MCTP1, GAS1, BIRC3, BQ186674, MAL, UBXD3, and WNT2B for the over-expressed genes, and GALNT14, TM4SF1, ZAR1, A_23_P10091, GLT8D2, RXFP1, CGNL1, AK094972, LRCH2, BM930757, ATP8A1, SOX9, SLC39A8, TMEM47, SLC10A4, SLC1A3, EDG7, ITGA2, PLCXD3, BF514799, SLC16A12, THC2208430, THC2182743, C4orf18, ANKRD38, CALCRL, and LOC152573 for the under-expressed genes; 2) contacting said cell-line with a test compound; 3) determining the expression level of said at least one gene; and, 4) selecting the compound which decreases the expression level of over-expressed genes and increases the expression level of under-expressed genes.

16. The method according to any one of claims 13 to 15, wherein the cell-line is a cancer cell-line.
